Europäisches Patentamt

European Patent Office

Office européen des brevets

(19)

(11) Publication number: **0 362 526 B1**

## EUROPEAN PATENT SPECIFICATION

(45) Date of publication of patent specification: **14.06.95**

(51) Int. Cl.⁶: **C12N 15/12**, C07K 14/705, A61K 38/17, C12P 21/02, A61K 35/12, C12Q 1/68, G01N 33/68, G01N 33/574

(21) Application number: **89115160.7**

(22) Date of filing: **17.08.89**

(54) The Alpha-subunit of the LFA-1 leukocyte adhesion receptor.

(30) Priority: **23.08.88 US 235227**
**09.03.89 US 321017**

(43) Date of publication of application:
**11.04.90 Bulletin 90/15**

(45) Publication of the grant of the patent:
**14.06.95 Bulletin 95/24**

(84) Designated Contracting States:
**AT BE CH DE ES FR GB GR IT LI LU NL SE**

(56) References cited:

**J. Cell. Biochem. 1987, suppl. o (11 part D), p. 272**

**NATURE, vol. 314, 11. April 1985, pp. 540-542, London, GB; T.A. SPRINGER et al.**

(73) Proprietor: **DANA FARBER CANCER INSTITUTE**
**44 Binney Street**
**Boston**
**Massachusetts 02115 (US)**

(72) Inventor: **Springer, Timothy Alan**
**28 Monadnock Road**
**Newton**
**Massachusetts 02109 (US)**
Inventor: **Larson, Richard**
**222, Calumnet Street**
**Boston**
**Massachusetts 02120 (US)**

(74) Representative: **Laudien, Dieter, Dr. et al**
**Boehringer Ingelheim Zentrale GmbH**
**ZA Patente**
**Postfach 200**
**D-55216 Ingelheim am Rhein (DE)**

Rank Xerox (UK) Business Services
(3.10/3.09/3.3.3)

EP 0 362 526 B1

**Description**

FIELD OF THE INVENTION:

The present invention relates to the leukocyte adhesion receptor LFA-1. The invention further pertains to the cloning of DNA sequences which encode the alpha-subunit of this molecule.

DESCRIPTION OF THE RELATED ART

The immune system is responsible for protecting an animal from foreign invaders, such as bacteria, viruses, etc. An excellent review of the defense system is provided by Eisen, H.W. ( In: Microbiology, 3rd Ed., Harper & Row, Philadelphia, PA (1980), pp. 290-295 and 381-418). The ability of the immune system to protect an animal against foreign invaders depends, in large measure, on the presence and function of blood cells known an leukocytes. The ability of leukocytes to provide such protein has been found to require that these cells adhere to cellular and extracellular substrates.

For example, leukocytes must be able to attach to endothelial cells so that they can migrate circulation to sites of ongoing inflammation. Furthermore, they must attach to antigen-presenting cells so that a normal immune response can occur. They must also be able to attach to appropriate target cells so that the lysis of virally-infected (or tumor) cells can occur. Furthermore, leukocytes must be able to attach to various activated proteins (such as iC3b-the activated form of the third component of complement) so that they may efficiently phagocytose and clear microbial and cellular debris. Thus, leukocyte adhesion is a requisite of a normally functioning host defense system. The inhibition of this defense system is desirable in cases such as transplantation, because the host "sees" the transplanted tissue as foreign and initiates an immune response to that tissue. Leukocyte adhesion is, therefore, also involved in the rejection of transplanted tissue and organs. Thus, an understanding of leukocyte adhesion may enable one to either augment an animal's ability to fight infection or suppress an animal's capacity to reject transplanted tissue.

Recently, leukocyte surface molecules involved in mediating leukocyte adhesion were identified using hybridoma technology. Briefly, monoclonal antibodies directed against human T-cells (Davignon, D., et al., Proc. Natl. Acad. Sci. USA 78: 4535-4539 (1981)) and mouse spleen cells (Springer, T., et al., Eur. J. Immunol. 9:-301-306 (1979)) were identified which bound to leukocyte surfaces and inhibited the attachment-related functions described above (Springer, T., et al., Fed. Proc. 44: 2660-2663 (1985)). The molecules which were recognized by these antibodies comprise a set of leukocyte adhesion receptors known as the "Lymphocyte Function-Associated Antigen-1 family" (or the "LFA-1 family") of adhesion receptor molecules.

The LFA-1 family of adhesion receptor molecules contains three highly related cell surface glycoproteins. These glycoproteins have been found to mediate cell-cell interactions in inflammation. The glycoproteins have been designated "LFA-1" (lymphocyte function-associated antigen-1), "Mac-1" and "p150,95." Whereas LFA-1 is found on the surfaces of most leukocytes (Springer, T.A., et al., Immunol. Rev. 68:111-135 (1982)), Mac-1 and p150,95 are found primarily on macrophages, granulocytes and other large granular lymphocytes (Springer, T.A., et al., Immunol. Rev. 68:111-135 (1982); Keizer, G., et al., Eur. J. Immunol. 15: 1142-1147 (1985)).

The LFA-1 glycoprotein family is composed of heterodimers, each containing an alpha-subunit which is non-covalently associated with a beta-subunit. The alpha-subunits of the family have been found to differ from one another and are designated CD11a, CD11b, and CD11c, respectively. The glycosylated alpha-subunits have approximate molecular weights of 180, 170, and 150 kd, respectively. In contrast, the beta-subunit of the LFA-1 family of adhesion receptors has been found to be identical, and to have a molecular weight of 95 kd (Sanchez-Madrid, F., et al., J. Exper. Med. 158:1785-1803 (1983); Keizer, G.D., et at., Eur. J. Immunol. 15: 1142-1147 (1985); Springer, T., Fed. Proc. 44:2660-2663 (1985); Sanchez-Madrid, F., et at., J. Exper. Med. 158:586-602 (1983)).

Although the alpha-subunits of the glycoproteins do not exhibit the extensive homology shared by the beta-subunits, close analysis of the alpha-subunits of the glycoproteins has revealed that there are substantial similarities between them. Reviews of the similarities between the alpha and beta-subunits of the adhesion molecule glycoprotein family are provided by Sanchez-Madrid, F., et at. (J. Exper. Med. 158:586-602 (1983); J. Exper. Med. 158:1785-1803 (1983); Miller, L.J., et al., J. Immunol. 138:2381-2383 (1987)).

Springer et al. (Nature 314:540-542(1985)) report the purification of LFA-1 alpha-subunit from murine lymphoma cells and obtained a N-terminal amino acid sequence 19 residues in length by amino acid sequencing. Larson et al. (J. Cell. Biochem. 1987, Suppl. O (11 part D), p.272) give a brief summary of the isolation of human LFA-1 and the attempts to isolate a respective cDNA clone. This publication, however,

EP 0 362 526 B1

does not comprise sufficient information in order to enable a skilled person to achieve these tasks.

The importance of the LFA-1 family of receptors was initially recognized in studies which showed the ability of monoclonal antibodies (which were capable of binding to either the specific alpha-subunits, or the common beta-subunit) to inhibit adhesion-dependent leukocyte functions (Sanchez-Madrid, F., et al., Proc. Natl. Acad.Sci. USA 79: 7389-7493 (1982); Beller, D.I., et al., J. Exper. Med. 156:1000-1009(1982)).

Recently, a group of individuals has been identified who are unable to express normal amounts of any member of the LFA-1 adhesion protein family on their leukocyte cell surfaces. This disease has been termed "Leukocyte Adhesion Deficiency" or "LAD" and is characterized by chronic and recurring infections, as well as other clinical symptoms (Anderson, D.C., et al., Fed. Proc. 44:2671-2677 (1985); Anderson, D.C., et al., J. Infect. Dis. 152:668-689 (1985)). Leukocytes from LAD patients display in vitro defects which were similar to those observed when leukocytes of normal individuals were antagonized by antibody specific for members of the LFA-1 family. LAD patients were found to be unable to mount a normal immune response. This failure was found to be due to inability of the leukocytes of LAD patients to adhere to cellular and extracellular substrates (Anderson, D.C., et al., Fed. Proc. 44:2671-2677.(1985); Anderson, D.C., et al., J. Infect. Dis. 152:668-689 (1985)). These studies show that inflammatory reactions are mitigated when leukocytes are unable to adhere in a normal fashion due to the lack of functional adhesion molecules on their cell surface.

Thus, in summary, the ability of leukocytes to maintain the health and viability of an animal requires that they be capable of adhering to other cells (such as endothelial cells) and proteins (such as iC3b). This adherence has been found to require contacts which involve specific receptor molecules present on the leukocyte surface of the leukocytes. These cell surface receptor molecules have been found to be highly related to one another. Humans whose leukocytes lack these cell surface receptor molecules exhibit chronic and recurring infections, as well as other clinical symptoms.

Since leukocyte adhesion is involved in the process through which foreign tissue is identified and rejected, an understanding of this process is of significant value in the fields of organ transplantation, tissue grafts, allergy and oncology.

SUMMARY OF THE INVENTION

The present invention relates to leukocyte cell surface adhesion receptor molecules, and in particular, to the cloning and expression of the human alpha-subunit of the LFA-1 receptor molecule through the use of recombinant DNA technology. The invention pertains to the adhesion molecule itself, to functional fragments of the molecule, to nucleic acid (i.e., DNA, and especially cDNA) capable of encoding these receptor molecules, and to plasmids which contain such nucleic acid sequences. The present invention additionally encompasses methods for producing the receptor molecules which employ recombinant DNA technology.

In detail, the invention pertains to human LFA-1 alpha-subunit, or a functional derivative thereof, substantially free of natural contaminants.

The invention further pertains to theabove LFA-1 alpha-subunit or the functional derivative thereof, which is additionally capable of binding to a molecule (such as ICAM-1, or LFA-1 beta-subunit) present on the surface of a cell.

The invention also includes the above LFA-1 alpha-subunit molecule, wherein the molecule contains at least one polypeptide selected from the group consisting of:

| | |
|---|---|
| a. P-P-R-A-G-R-H; | h. G-V-D-V-Q-D-G-E-I-E; |
| b. I-I-T-D-G-E-A; | i. D-I-N-G-D-G-L-V-D-V; |
| c. D-W-A-G-G-F-L; | j. V-K-D-L-E-G-D-G-L-A; |
| d. S-Q-V-Q-T-I-H; | k. T-Y-L-S-G-L; |
| e. R-H-G-G-L-S-P; | l. Y-I-I-G-I-G-K; |
| f. M-S-C-T-D-F-S; | m. I-E-G-T-Q-V-L-S-Q; and |
| g. R-L-L-S-R-A-L; | n. P-S-I-H-N-I-P. |

The invention includes a recombinant DNA molecule capable of expressing either the human LFA-1 alpha-subunit or a functional derivative thereof.

The invention also provides a method for recovering LFA-1 alpha-subunit in substantially pure form which comprises the steps:

(a) solubilizing LFA-1 alpha-subunit from the membranes of cells expressing LFA-1 alpha-subunit, to form a solubilized LFA-1 alpha-subunit preparation,

3

EP 0 362 526 B1

(b) introducing the solubilized LFA-1 alpha-subunit preparation to an affinity matrix, the matrix containing immobilized antibody capable of binding to LFA-1 alpha-subunit,
(c) permitting the LFA-1 alpha-subunit to bind to the antibody of the affinity matrix,
(d) removing from the matrix any compound incapable of binding to the antibody and
(e) recovering the LFA-1 alpha-subunit in substantially pure form by eluting the LFA-1 alpha-subunit from the matrix.

The invention also provides a method for treating inflammation resulting from a response of the specific defense system (such as delayed-type hypersensitivity; graft versus host disease, tissue graft rejection, organ transplant rejection, autoimmune diseases such as lupus erythemotosus, autoimmune thyroiditis, experimental allergic encephalomyelitis (EAE), multiple sclerosis, some forms of diabetes, Reynaud's syndrome, rheumatoid arthritis, etc.) in a mammalian subject which comprises providing to a subject in need of such treatment an amount of an anti-inflammatory agent sufficient to suppress the inflammation; wherein the anti-inflammatory agent is selected from the group consisting of: the LFA-1 alpha-subunit, and a functional derivative of LFA-1 alpha-subunit.

The invention further includes the above method, which additionally comprises the co-administration of an agent selected from the group consisting of: the LFA-1 beta-subunit, and a functional derivative of the LFA-1 beta-subunit.

The invention also pertains to a method of suppressing the metastasis of a hematopoietic tumor cell, the cell requiring a functional member of the human LFA-1 family for migration, which method comprises providing to a patient in need of such treatment an amount of an anti-inflammatory agent sufficient to suppress the metastasis; wherein the anti-inflammatory agent being selected from the group consisting of: LFA-1 alpha-subunit, and a functional derivative of the LFA-1 alpha-subunit.

The invention also pertains to a method of suppressing the growth of a human LFA-1 alpha-subunit-expressing tumor cell which comprises providing a patient in need of such treatment an amount of a toxin sufficient to suppress the growth, the toxin being selected from the group consisting of a toxin-derivatized LFA-1 alpha-subunit, and a toxin-derivatized functional derivative of a member of the LFA-1 alpha-subunit.

The invention also pertains to a method of diagnosing the presence and location of inflammation resulting from a response of the specific defense system in a mammalian subject suspected of having the inflammation which comprises:

(a) administering to the subject a composition containing a detectably labeled human LFA-1 alpha-subunit capable of identifying a cell which expresses ICAM-1, or another natural ligand of LFA-1, and
(b) detecting the LFA-1 alpha-subunit.

The invention also pertains to a method of diagnosing the presence and location of inflammation resulting from a response of the specific defense system in a mammalian subject suspected of having the inflammation which comprises:

(a) incubating a sample of tissue of the subject with a composition containing a detectably labeled human LFA-1 alpha-subunit capable of identifying a cell which expresses ICAM-1, or another natural ligand of LFA-1, and
(b) detecting the LFA-1 alpha-subunit.

The invention also pertains to a method of diagnosing the presence and location of inflammation resulting from a response of the specific defense system in a mammalian subject suspected of having the inflammation which comprises:

(a) incubating a sample of tissue of the subject with a composition containing a nucleic acid molecule capable of binding to a molecule selected from the group consisting of a DNA sequence of human LFA-1 alpha-subunit, and an mRNA sequence of a gene for LFA-1 alpha-subunit, wherein the binding is capable of identifying a cell which is capable of binding ICAM-1, or another natural ligand of LFA-1, and
(b) detecting the nucleic acid molecule.

The invention also pertains to a method of diagnosing the presence and location of inflammation resulting from a response of the specific defense system in a mammalian subject suspected of having the inflammation which comprises:

(a) incubating a sample of tissue of the subject with a composition containing a detectably labeled LFA-1 alpha-subunit capable of identifying a cell which expresses ICAM-1, or another natural ligand of LFA-1, and
(b) detecting the LFA-1 alpha-subunit.

The invention also pertains to a method of diagnosing the presence and location of a tumor cell which expresses ICAM-1 or another natural ligand of LFA-1, in a subject suspected of having such a cell, which comprises:

4

(a) administering to the subject a composition containing a detectably labeled binding ligand capable of binding to the ICAM-1, or the other natural ligand of LFA-1, the ligands being selected from the group consisting of LFA-1 alpha-subunit and a functional derivative of LFA-1 alpha-subunit, and

(b) detecting the binding ligand.

The invention also pertains to a method of diagnosing the presence and location of a tumor cell which expresses ICAM-1 of another natural ligand of LFA-1, in a subject suspected of having such a cell, which comprises:

(a) incubating a sample of tissue of the subject in the presence of a composition containing a detectably labeled binding ligand capable of binding to the ICAM-1, or the other natural ligand of LFA-1, the ligands being selected from the group consisting of LFA-1 alpha-subunit and a functional derivative of LFA-1 alpha-subunit, and

(b) detecting the binding ligand which is bound to the ICAM-1 present in the sample of tissue.

## BRIEF DESCRIPTION OF THE FIGURES

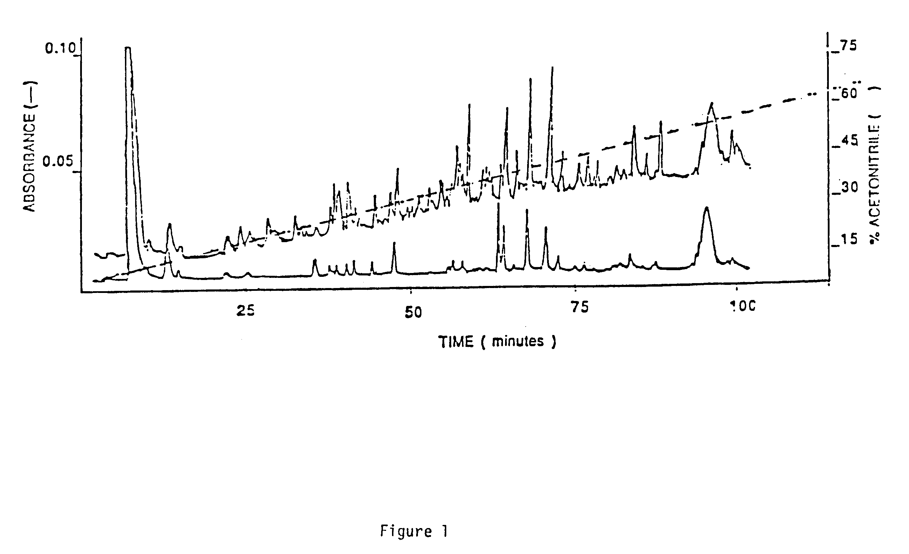

Figure 1 shows a profile of the reverse-phase HPLC separation of the LFA-1 alpha-subunit tryptic peptides. Elution was monitored by optical density at 280 nm (lower profile) and 214 nm (upper profile). The peptides indicated by a dot were subjected to protein micro-sequencing. The line across the profile indicates the percentage of acetonitrile.

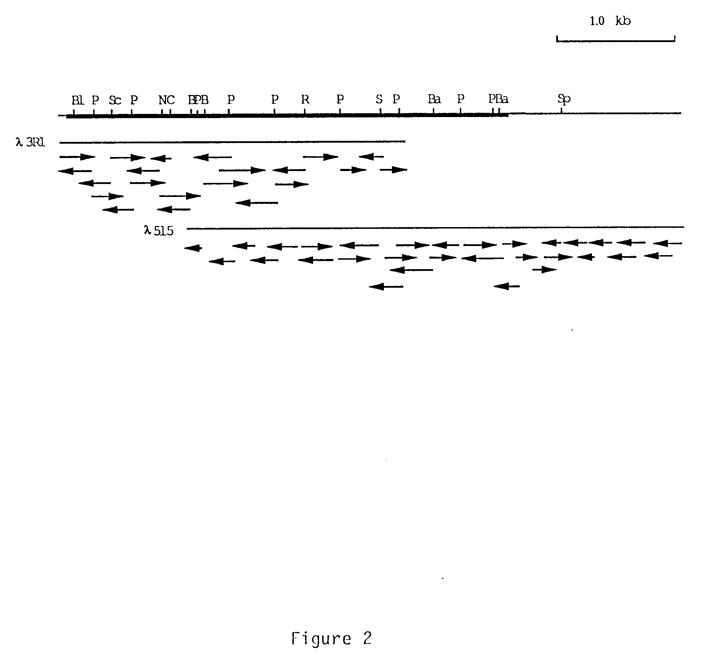

Figure 2 shows a restriction map of LFA-1 alpha-subunit cDNA clones. Restriction sites are Bal I (B1), Bam HI (B), Bgl II (Bg), Cla I (C), Eco RI (R), Hinc II (H), Nru I (N), Pst I (P), Sca I (Sc), and Sma I (S). Arrows indicating the sequencing strategy are shown.

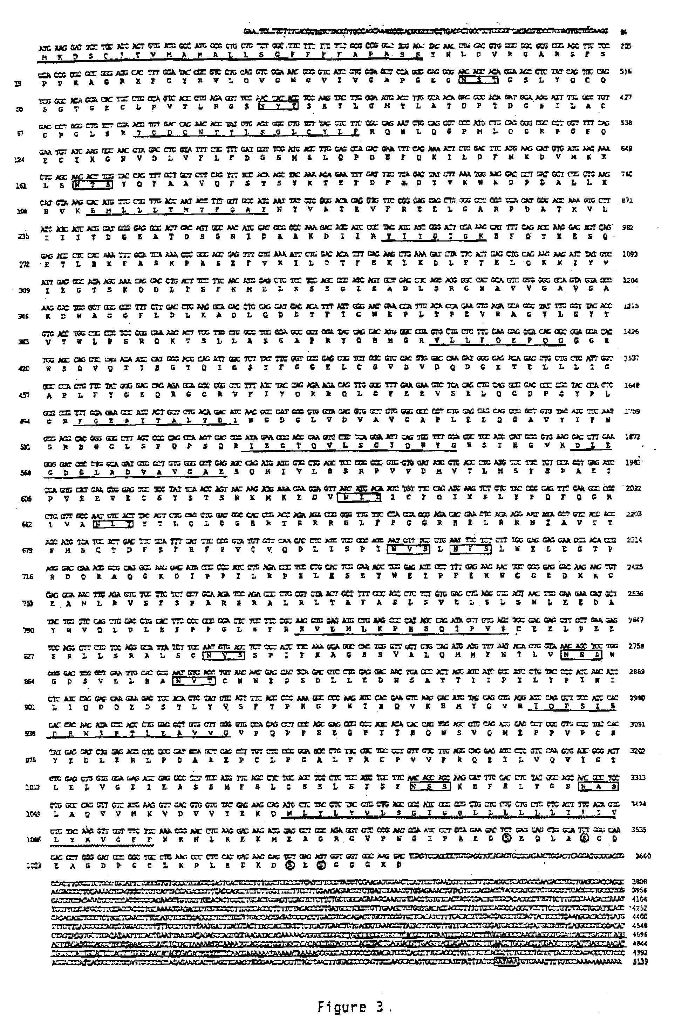

Figure 3 shows the nucleotide and derived amino acid sequence of the LFA-1 alpha-subunit. The sequences of the tryptic peptides and the transmembrane region are underlined with a thick and a shaded line, respectively. The putative serine phosphorylation sites are circled. The nucleotides in the 3′-untranslated region that are underlined correspond to an Alu1 sequence.

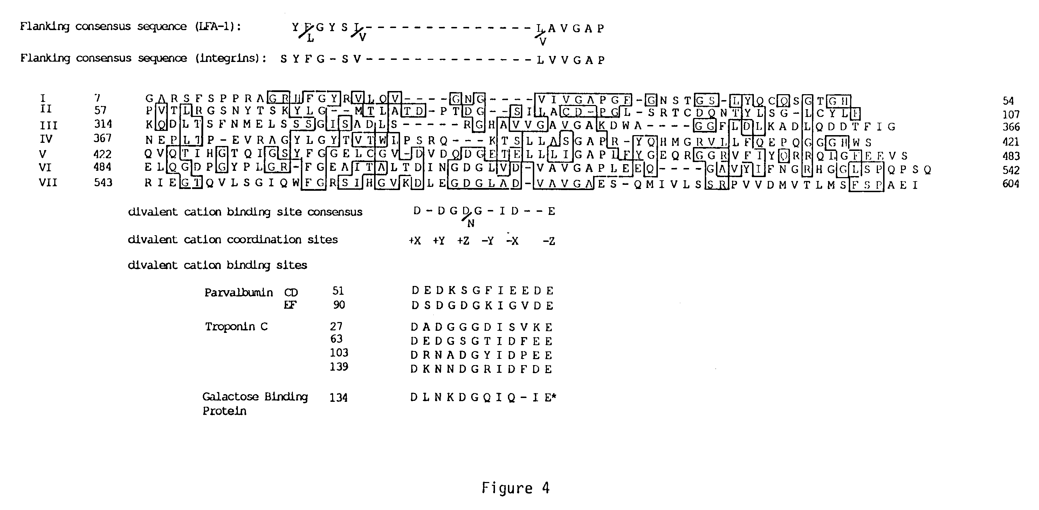

Figure 4 shows an alignment of the internal repeats of the LFA-1 alpha-subunit. The concensus flanking sequence is shown above the aligned repeats, while the concensus divalent binding site and previously described binding sites are shown below. An asterisk indicates that more then one oxygen is involved in cation binding.

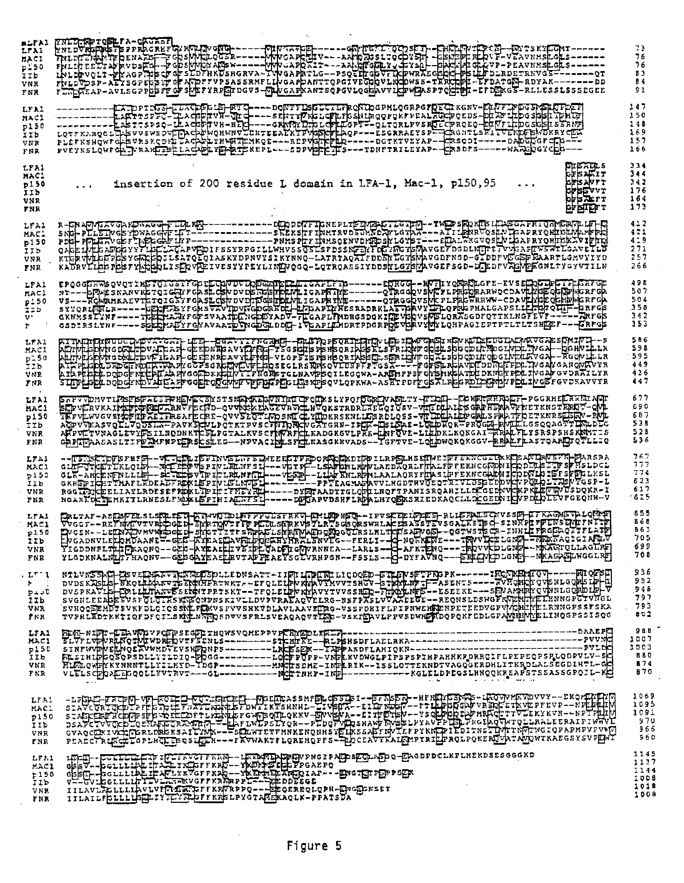

Figure 5 shows an alignment of the human LFA-1 alpha-subunit with the other members of the integrin supergene family and the N-terminal of the murine LFA-1 alpha-subunit. The residues common to LFA-1 and at least one integrin are boxed. The area of the leukocyte insert is notable. The boundaries of the homologous repeats containing the divalent cation binding sites are indicated with triangles. The protease cleavage site in the ECM receptor alpha-subunits are indicated with arrows. The transmembrane region is underlined.

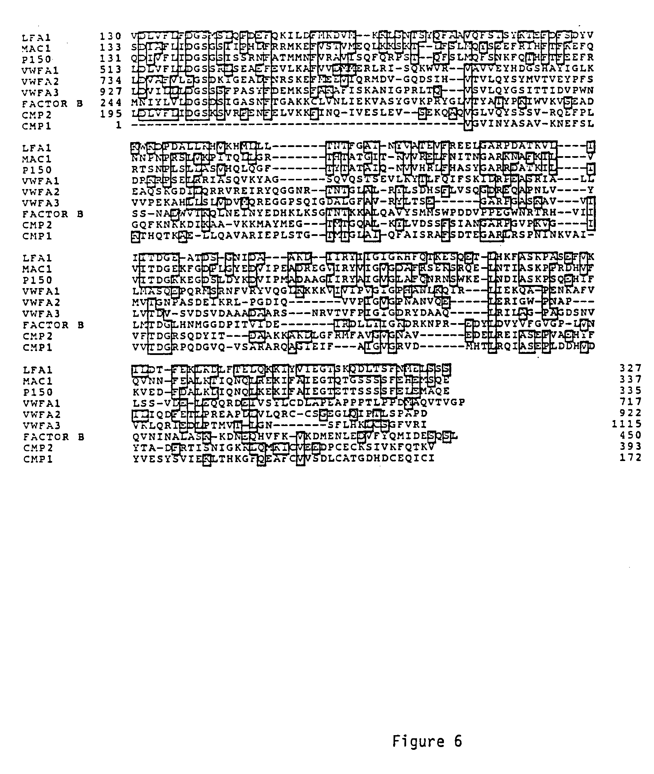

Figure 6 shows an alignment and comparison of the LFA-1 alpha-subunit L domain with the homologous domains in von Willebrand's Factor (vWF), Factor B, and CMP.

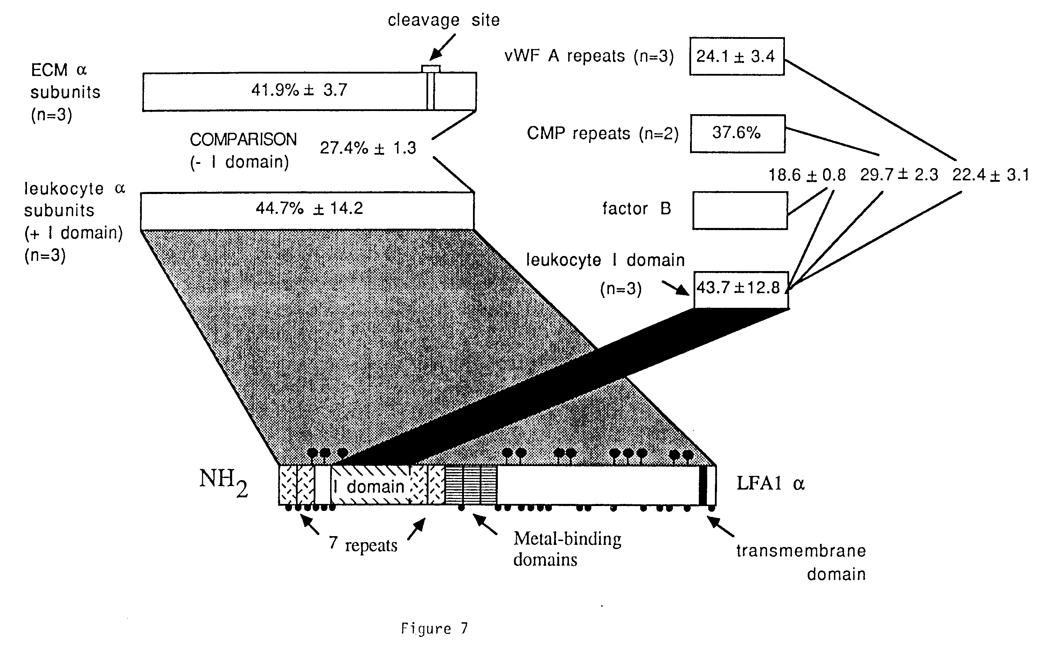

Figure 7 shows a schematic representation of the evolutionary relationships of the domains homologous to the L domain.

## DESCRIPTION OF THE PREFERRED EMBODIMENTS

### I. The Nature of the Leukocyte Adhesion Proteins of the LFA-1 Family

The three leukocyte adhesion proteins Mac-1, p150,95 and LFA-1 differ in function and in expression on leukocyte subpopulations. Mac-1 and p150,95 are expressed on neutrophils, and monocytes (Springer, T.A., et al., In: Biochemistry of Macrophages (CIBA Symposium 118); Pitman, London, pp. 102-126 (1986)). During differentiation of blood monocytes into tissue macrophages, expression of p150,95 is greatly increased and Mac-1 expression is decreased (Schwarting, R., et al., Blood 65:974-983 (1985); Hogg, N., et al., Eur. J. Immunol. 16:240-248 (1986)). p150,95 is also expressed on certain types of activated T and B lymphocytes, but is not expressed on these cells in the blood (Kaligaris-Cappio, F., et al., Blood 66:1035-1042 (1985); Miller, L.J., et al., J. Immunol. 137:2891-2900 (1986); Keizer, G.D., et al., J. Immunol. 138:3130-3136 (1987)).

Mac-1 and p150,95 are expressed in an intracellular, vesicular compartment in circulating neutrophils and monocytes which is mobilized to the cell surface by inflammatory mediators (Todd, R.F., et al., J. Clin. Invest. 74:1280-1290 (1984); Springer, T.A., et al., In: Biochemistry of Macrophages (CIBA Symposium 118), Pitman, London, pp. 102-126 (1986); Lanier, L.L., et al., Eur. J. Immunol. 15:713-718 (1985); Yancey, K.B., et al., J. Immunol. 135:465-470 (1985)). This mobilization correlates with increased adhesiveness (Anderson, D.C., et al., Ann. Rev. Med. 38:175-194 (1987)).

Monoclonal antibodies to Mac-1 or p150,95 inhibit neutrophil aggregation and adherence to endothelial cells, protein-coated surfaces, bacteria, protozoan parasites, and fungi (Harlan, J.M. et al., Blood 66:167-178 (1985); Springer, T.A., et al., In: Biochemistry of Macrophages (CIBA Symposium 118), Pitman, London pp. 102-126 (1986); Dana, N., et al., J. Immunol. 137:3259 (1986); Bullock, W.D., et al., J. Exper. Med. 165:195-210 (1987); Mosser, D.M., et al., J. Immunol. 135:2785-2789 (1985)).

Mac-1 is also a receptor for the complement component iC3b (Beller, D.I., et al., J. Exper. Med. 156:1000-1009 (1982)). Detergent-soluble Mac-1 and p150,95 have been shown to be able to bind to iC3b-Sepharose (Micklem, K.J., et al., Biochem. J. 231:233-236 (1985)).

LFA-1 is present on all leukocytes except a subset of macrophages. Monoclonal antibody blocking studies have shown that LFA-1 is important in T-lymphocyte-mediated killing, T helper lymphocyte responses, natural killing, and antibody-dependent killing (Springer, T.A., et al., An. Rev. Immunol. 5:223-252 (1987)). Adhesion to the target cell is a step which is blocked by antibodies against LFA-1. Functional studies have suggested that LFA-1 interacts with several ligands, one of which is ICAM-1 (Rothlein, R., et al., J. Immunol. 137:1270-1274 (1986)).

Some cytotoxic T lymphocyte clones have been found to express similar quantities of p150,95 and LFA-1. Monoclonal antibodies to the LFA-1 and p150,95 alpha-subunits inhibit killing by such CTL clones to similar extents and are additive in their inhibitory effects (Keizer, G.D., et al., J. Immunol. 138:3130-3136 (1987)). Furthermore, antibodies to p150,95 alpha-subunits have been shown to inhibit monocyte attachment to endothelium (Keizer, G.D., et al., Eur. J. Immunol. 17:1317-1322 (1987)).

II. Cloning of the LFA-1 Alpha-subunit

Any of a variety of methods can be used to clone the LFA-1 alpha-subunit gene. One such method entails analyzing a shuttle vector library of cDNA inserts (derived from a LFA-1 alpha-subunit expressing cell) for the presence of an insert which contains the LFA-1 alpha-subunit gene. Such an analysis may be conducted by transfecting cells with the vector, and then assaying for LFA-1 alpha-subunit expression. A preferred method for cloning the LFA-1 alpha-subunit gene entails determining the amino acid sequence of the LFA-1 alpha-subunit molecule, or tryptic peptides of the molecule. To accomplish this task, LFA-1 alpha-subunit molecules are preferably purified from producer cells by monoclonal antibody affinity chromatography and isolated by preparative sodium dodecyl sulfate-polyacrylamide gel electrophoresis ("SDS-PAGE") and electroelution (Miller, L.J., et al., J. Immunol. 138:2381-2383 (1987), which reference herein is incorporated by reference). The alpha-subunit molecules are fragmented as with cyanogen bromide, or with proteases such as papain, chymotrypsin, or trypsin (Oike, Y., et al., J. Biol. Chem. 257:9751-9758 (1982); Liu, C., et al., Int. J. Pept. Protein Res. 21:209-215 (1983)). Preferably, the alpha-subunit is proteolytically digested with trypsin. The resulting peptides are separated by reverse-phase HPLC and subjected to amino acid sequencing. To accomplish this task, the protein is, preferably, analyzed by automated sequenators. Although it is possible to determine the entire amino acid sequence of the LFA-1 alpha-subunit, it is preferably to determine the sequence of peptide fragments of the molecule. A preferred source of the LFA-1 alpha-subunit is the SKW3 cell line.

The sequence of amino acid residues in a peptide is designated herein either through the use of their commonly employed three-letter designations or by their single-letter designations. A listing of these three-letter and one-letter designations may be found in textbooks such as Biochemistry, Lehninger, A., Orth Publishers, New York, NY (1970). When such a sequence is listed vertically, the amino terminal residue is intended to be at the top of the list, and the carboxy terminal residue of the peptide is intended to be at the bottom of the list. Similarly, when listed horizontally, the amino terminus is intended to be on the left end whereas the carboxy terminus is intended to be at the right end.

The residues of amino acids in a peptide may be separated by hyphens. Such hyphens are intended solely to facilitate the presentation of a sequence. As a purely illustrative example, the amino acid sequence designated:

-Gly-Ala-Ser-Phe-

indicates that an Ala residue is linked to the carboxy group of Gly, and that a Ser residue is linked to the carboxy group of the Ala residue and to the amino group of a Phe residue. The designation further indicates that the amino acid sequence contains the tetrapeptide Gly-Ala-Ser-Phe. The designation is not intended to limit the amino acid sequence to this one tetrapeptide, but is intended to include (1) the tetrapeptide having one or more amino acids linked to either its amino or carboxy end, (2) the tetrapeptide having one or more amino acid residues linked to both its amino and its carboxy ends, (3) the tetrapeptide having no additional

6

amino acid residues.

Once one or more suitable peptide fragments have been sequenced, the DNA sequences capable of encoding them are examined. because the genetic code is degenerate, more than one codon may be used to encode a particular amino acid (Watson, J.D., In: Molecular Biology of the Gene, 3rd Ed., W.A. Benjamin, Inc., Menlo Park, CA (1977), pp. 356-357). The peptide fragments are analyzed to identify sequences of amino acids which may be encoded by oligonucleotides having the lowest degree of degeneracy. This is preferably accomplished by identifying sequences that contain amino acids which are encoded by only a single codon.

Although occasionally an amino acid sequences may be encoded by only a single oligonucleotide, frequently the amino acid sequence may be encoded by any of a set of similar oligonucleotides. Important, whereas all of the members of this set contain oligonucleotides which are capable of encoding the peptide fragment and, thus, potentially contain the same oligonucleotide sequence as the gene which encodes the peptide fragment, only one member of the set contains the nucleotide sequence that is identical to the nucleotide sequence of the gene. Because this member is present within the set, and is capable of hybridizing to DNA even in the presence of the other members of the set, it is possible to employ the unfractionated set of oligonucleotides in the same manner in which one would employ a single oligonucleotide to clone the gene that encodes the peptide.

A suitable oligonucleotide, or set of oligonucleotides, which is capable of encoding a fragment of the LFA-1 alpha-subunit gene (or which is complementary to such an oligonucleotide, or set of oligonucleotides) is identified (using the above-described procedure), synthesized, and hybridized by means well known in the art, against a DNA or, more preferably, a cDNA preparation derived from human cells which are capable of expressing the LFA-1 alpha-subunit gene. Techniques of nucleic acid hybridization are disclosed by Maniatis, T., et al. (In: Molecular Cloning, A Laboratory Manual, Cold Spring Harbor Laboratories, Cold Spring Harbor, NY (1982)), and by Haymes, B.D., et al. (In: Nucleic Acid Hybridization, A Practical Approach, IRL Press, Washington, DC (1985)), which references are herein incorporated by reference. The source of DNA or cDNA used will preferably have been enriched for LFA-1 alpha-subunit sequences. Such enrichment can most easily be obtained from cDNA obtained by extracting RNA from cells which produce high levels of the LFA-1 alpha-subunit.

Techniques such as, or similar to, those described above have successfully enabled the cloning of genes for human aldehyde dehydrogenases (Hsu, L.C., et al., Proc. Natl. Acad. Sci. USA 82:3771-3775 (1985)), fibronectin (Suzuki, S., et al., Eur. Mol. Biol. Organ. J. 4:2519-2524 (1985)), the human estrogen receptor gene (Walter, P., et al., Proc. Natl. Acad. Sci. USA 82:7889-7893 (1985)), tissue-type plasminogen activator (Pennica, D., et al., Nature 301:214-221 (1983)) and human term placental alkaline phosphatase complementary DNA (Kam, W., et al., Proc. Natl. Acad. Sci. USA 82:8715-8719 (1985)).

Using the genetic code (Watson, J.D., In: Molecular Biology of the Gene, 3rd Ed., W.A. Benjamin, Inc., Menlo Park, CA (1977)), one or more different oligonucleotides can be identified, each of which would be capable of encoding the LFA-1 alpha-subunit tryptic peptides. The probability that a particular oligonucleotide will, in fact, constitute the actual LFA-1 alpha-subunit-encoding sequence can be estimated by considering abnormal base pairing relationships and the frequency with which a particular codon is actually used (to encode a particular amino acid) in eukaryotic cells. Such "codon usage rules" are disclosed by Lathe, R., et al., J. Molec. Biol. 183:1-12 (1985). Using the "codon usage rules" of Lathe, a single oligonucleotide, or a set of oligonucleotides, that contains a theoretical "most probable" nucleotide sequence capable of encoding the LFA-1 alpha-subunit tryptic peptide sequences is identified.

The oligonucleotide, or set of oligonucleotides, containing the theoretical "most probable" sequence capable of encoding the LFA-1 alpha-subunit fragments is used to identify the sequence of a complementary oligonucleotide or set of oligonucleotides which is capable of hybridizing to the "most probable" sequence, or set of sequences. An oligonucleotide containing such a complementary sequence can be employed as a probe to identify and isolate the LFA-1 alpha-subunit gene (Maniatis, T., et al., Molecular Cloning A Laboratory Manual, Cold Spring Harbor Press, Cold Spring Harborn, NY (1982).

Thus, in summary, the actual identification of LFA-1 alpha-subunit peptide sequences permits the identification of a theoretical "most probable" DNA sequence, or a set of such sequences, capable of encoding such a peptide. By constructing an oligonucleotide complementary to this theoretical sequence (or by constructing a set of oligonucleotides complementary to the set of "most probable" oligonucleotides), one obtains a DNA molecule (or set of DNA molecules), capable of functioning as a probe to identify and isolate the LFA-1 alpha-subunit gene.

Single stranded oligonucleotide molecules complementary to the "most probable" LFA-1 alpha-subunit tryptic peptide encoding sequences were synthesized using procedures which are well known to those of ordinary skill in the art (Belagaje, R., et al., J. Biol. Chem. 254:5765-5780 (1979); Maniatis, T., et al., In:

Molecular Mechanisms in the Control of Gene Expression, Nierlich, D.P., et al., Eds., Acad. Press, NY (1976); Wu, R., et al., Prog. Nucl. Acid Res. Molec. Biol. 21:101-141 (1978); Khorana, R.G., Science 203:614-625 (1979)). Additionally, DNA synthesis may be achieved through the use of automated synthesizers.

It is possible to clone the LFA-1 alpha-subunit gene from eukaryotic DNA preparations suspected of containing this gene. To identify and clone the gene which encodes the LFA-1 alpha-subunit protein, a DNA, or more preferably a cDNA, library is screened for its ability to hybridize with the oligonucleotide probes described above. Suitable DNA preparations (such as human genomic DNA) are enzymatically cleaved, or randomly sheared, and ligated into recombinant vectors. The ability of these recombinant vectors to hybridize to the above-described oligonucleotide probes is then measured. Procedures for hybridization are disclosed, for example, in Maniatis, T., Molecular Cloning A Laboratory Manual, Cold Spring Harbor Press, Cold Spring Harbor, NY (1982) or in Haymes, B.T., et al., Nucleic Acid Hybridization a Practical Approach, IRL Press, Oxford, England (1985). Vectors found capable of such hybridization are then analyzed to determine the extent and nature of the LFA-1 alpha-subunit sequences which they contain. Based purely on statistical considerations, a gene such as that which encodes the LFA-1 alpha-subunit molecule could be unambiguously identified (via hybridization screening) using an oligonucleotide probe having only 18 nucleotides.

The cloned LFA-1 alpha-subunit gene, obtained through the method described above, may be operably linked to an expression vector, and introduced into prokaryotic or eukaryotic cells to produce the LFA-1 alpha-subunit protein. Techniques for such manipulations are disclosed by Maniatis, T., et al., supra, and are well known in the art.

III. The Expression of the LFA-1 Alpha-subunit

The present invention derives, in part, from the discovery of the cDNA sequence which encodes the alpha-subunit of the LFA-1 molecule. By operably linking this sequence (or a fragment of this sequence) to a functional promoter, it is possible to direct the expression of the alpha-subunit of LFA-1 (or a functional derivative thereof) in a cell, or organism.

A nucleic acid molecule, such as DNA, is said to be "capable of expressing" a polypeptide if it contains nucleotide sequences which contain transcriptional and translational regulatory information and such sequences are "operably linked" to nucleotide sequences which encode the polypeptide. An operable linkage is a linkage in which the regulatory DNA sequences and the DNA sequence sought to be expressed are connected in such a way as to permit gene expression. The precise nature of the regulatory regions needed for gene expression may vary from organism to organism, but shall in general include a promoter region which, in prokaryotes, contains both the promoter (which directs the initiation of RNA transcription) as well as the DNA sequences which, when transcribed into RNA, will signal the initiation of protein synthesis. Regulatory regions in eukaryotic cells will in general include a promoter region sufficient to direct the initiation of RNA synthesis.

Two DNA sequences (such as a promoter region sequence and a LFA-1 alpha-subunit-encoding sequence) are said to be operably linked if the nature of the linkage between the two DNA sequences does not (1) result in the introduction of a frame-shift mutation, (2) interfere with the ability of the promoter region sequence to direct the transcription of the LFA-1 alpha-subunit-encoding sequence, or (3) interfere with the ability of the LFA-1 alpha-subunit-encoding sequence to be transcribed by the promoter region sequence. Thus, a promoter region would be operably linked to a DNA sequence if the promoter were capable of effecting transcription of that DNA sequence.

The present invention encompasses the expression of the LFA-1 alpha-subunit (or a functional derivative thereof) in either prokaryotic or eukaryotic cells. To express the LFA-1 alpha-subunit (or a functional derivative thereof) in a prokaryotic cell (such as, for example, E. coli, B. subtilis, Pseudomonas, Streptomyces, etc.), it is necessary to operably link the LFA-1 alpha-subunit-encoding sequence to a functional prokaryotic promoter. Such promoters may be either constitutive or, more preferably, regulatable (i.e., inducible or derepressible). Examples of constitutive promoters include the int promoter of bacteriophage λ, the bla promoter of the β-lactamase gene of pBR322, and the CAT promoter of the chloramphenicol acetyl transferase gene of pPR325, etc. Examples of inducible prokaryotic promoters include the major right and left promoters of bacteriophage λ ($P_L$ and $P_R$), the tro, recA, lacZ, lacI, and gal promoters of E. coli, the α-amylase (Ulmanen, I., et al., J. Bacteriol. 162:176-182 (1985)) and the σ-28-specific promoters of B. subtilis (Gilman, M.Z., et al., Gene 32:11-20 (1984)), the promoters of the bacteriophages of Bacillus (Gryczan, T.J., In: The Molecular Biology of the Bacilli, Academic Press, Inc., NY (1982)), and Streptomyces promoters (Ward, J.M., et al., Mol. Gen. Genet. 203:468-478 (1986)). Prokaryotic promoters are reviewed by Glick, B.R., (J. Ind. Microbiol. 1:277-282 (1987)); Cenatiempo, Y. (Biochimie 68:505-516 (1986)); and Gottesman, S. (Ann. Rev. Genet. 18:415-442 (1984)).

Proper expression in a prokaryotic cell requires the presence of a ribosome binding site upstream of the gene-encoding sequence. Such ribosome binding sites are disclosed, for example, by Gold, L., et al. - (Ann. Rev. Microbiol. 35:365-404 (1981)).

If expression is desired in a eukarotic cell, such as yeast, fungi, mammalian cells, or plant cells, then it shall be necessary to employ a promoter capable of directing transcription in such a eukaryotic host. Preferred eukaryotic promoters include the promoter of the mouse metallothionein I gene (Hamer, D., et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London) 290:304-310 (1981)); the yeast gal4 gene promoter (Johnston, S.A., et al., Proc. Natl. Acad. Sci. (USA) 79:6971-6975 (1982); Silver, P.A., et al., Proc. Natl. Acad. Sci. (USA) 81:5951-5955 (1984)).

As is widely known, translation of eukaryotic mRNA is initiated at the codon which encodes the first methionine. For this reason, it is preferable to ensure that the linkage between a eukaryotic promoter and a DNA sequence which encodes the LFA-1 alpha-subunit (or a functional derivative thereof) does not contain any intervening codons which are capable of encoding a methionine (i.e., AUG). The presence of such codons results either in a formation of a fusion protein (if the AUG codon is in the same reading frame as the LFA-1 encoding DNA sequence) or a frame-shift mutation (if the AUG codon is not in the same reading frame as the LFA-1 encoding sequence).

A DNA sequence which encodes the LFA-1 protein (or a functional derivative thereof) when operably linked to a functional promoter is preferably introduced into a recipient cell by any of a variety of suitable means: transformation, transfection, conjugation, protoplast fusion, electroporation, etc.

The LFA-1 alpha-subunit-encoding sequence and an operably linked promoter may be introduced into a recipient cell either as a non-replicating DNA (or RNA) molecule, which may either be a linear molecule or, more preferably, a closed covalent circular molecule. Since such molecules are incapable of autonomous replication, the expression of the LFA-1 alpha-subunit polypeptide may occur through the transient expression of the introduced sequence. Alternatively, permanent expression may occur through the integration of the introduced sequence into the host chromosome.

Preferably, the introduced sequence will be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species. Preferred prokaryotic vectors include plasmids such as those capable of replication in E. coli (such as, for example, pBR322, ColE1, pSC101, pACYC 184, πVX. Such plasmids are, for example, disclosed by Maniatis, T., et al. (In: Molecular Cloning, A Laboratory Manual, Cold Spring Harbor Press, Cold Spring Harbor, NY (1982)). Bacillus plasmids include pC194, pC221, pT127, etc. Such plasmids are disclosed by Gryczan, T. (In: The Molecular Biology of the Bacilli, Academic Press, NY (1982), pp. 307-329). Suitable Streptomyces plasmids include pIJ101 (Kendall, K.J., et al., J. Bacteriol. 169:4177-4183 (1987)), and streptomyces bacteriophages such as φC31 (Chater, K.F., et al., In: Sixth International Symposium on Actinomyceteles Biology, Akademiai Kaido, Budapest, Hungary (1986), pp. 45-54). Pseudomonas plasmids are reviewed by John, J.F., et al. (Rev. Infect. Dis. 8:693-704 (1986)), and Izaki, K. (Jpn. J. Bacteriol. 33:729-742 (1978)).

Preferred eukaryotic plasmids include BPV, vaccinia, SV40, 2-micron circle, etc., or their derivatives. Such plasmids are well known in the art (Botstein, D., et al., Miami Wntr. Symp. 19:265-274 (1982); Broach, J.R., In: The Molecular Biology of the Yeast Saccharomyces: Life Cycle and Inheritance, Cold Spring Harbor Laboratory, Cold Spring Harbor, NY, p. 445-470 (1981); Broach, J.R., Cell 28:203-204 (1982); Bollon, D.P., et al., J. Clin. Hematol. Oncol. 10:39-48 (1980); Maniatis, T., In: Cell Biology: A Comprehensive Treatise, Vol. 3. Gene Expression, Academic Press, NY, pp. 563-608 (1980)).

IV. Uses of the LFA-1 Alpha-subunit, or Fragments Thereof

The present invention provides the nucleic acid and protein sequences of the alpha-subunit of the LFA-1 receptor molecule. This discovery permits the use of recombinant DNA technology to produce the LFA-1 alpha-subunit molecule. As discussed further below, one embodiment of the present invention pertains to the use of the alpha-subunit of the LFA-1 molecule by itself, as an anti-inflammatory agent. In one preferred embodiment, the alpha-subunit of the LFA-1 molecule will be used in combination with its beta-subunit. Such a combination may be produced using a variety of methods. For example, the beta-subunit of LFA-1 may be produced separately from the LFA-1 alpha-subunit, and the two molecules may then be mixed together. It is, however, preferable to produce both the alpha and beta-subunits of LFA-1 in the same host cell in order to facilitate their self-assembly into the LFA-1 heterodimer receptor molecule. The beta-subunit of LFA-1 (which is common to LFA-1, and Mac-1) may be produced either by chemical synthesis, or by recombinant DNA techniques (Kishimoto, T.K., et al., Cell 48:681-690 (1987)). The cloning of the beta-subunit of LFA-1 is further disclosed in commonly assigned, co-pending U.S. patent application Serial No. 019,440, filed on February 26, 1987, which application is herein incorporated by reference.

One aspect of the present invention relates to the discovery of the nucleic acid and protein sequences of the alpha-subunit of the LFA-1 receptor molecule. This discovery permits the use of recombinant DNA technology to produce functional derivatives of the LFA-1 alpha-subunit which may function as antagonists of cellular adhesion. As used herein, an "antagonist of cellular adhesion" is meant to refer to any molecule capable of inhibiting the process of cell-cell or cell-substrate adhesion. It is possible to determine whether a particular compound is an antagonist by performing an assay of leukocyte aggregation to endothelial cells. Suitable assays of leukocyte aggregation are disclosed, for example, (Rothlein, R. et al. J. Exper. Med. 163:1132-1149 (1986)) which reference is herein incorporated by reference. Antagonists of leukocyte aggregation may be employed as anti-inflammatory agents.

As used herein, a "functional derivative" of the alpha-subunit of LFA-1 is a compound which possesses a biological activity (either functional or structural) that is substantially similar to a biological activity of the alpha-subunit of LFA-1. Examples of biological activities include the ability to bind the ICAM-1, another natural ligand of the LFA-1 molecule, or the β-subunit of the LFA family of glycoproteins. Such binding would inhibit adhesion related events such as lymphocyte attachment to endothelial cells, antigen presenting cells or target cells. A molecule is said to be "substantially similar" to another molecule if both molecules have substantially similar structures or if both molecules possess a similar biological activity. The "functional derivatives: of the alpha-subunit of LFA-1 include both "fragments" and "variants" of the LFA-1 alpha-subunit. The term "fragment of the alpha-subunit of LFA-1" is meant to refer to any polypeptide subset of that molecule. The term "variant of the alpha-subunit of LFA-1" is meant to refer to a molecule substantially similar in structure to either the entire molecule, or to a fragment thereof provided that the "variant" has at least one biological activity that is either similar to an activity of the alpha-subunit of LFA-1 or inhibitory to an activity of LFA-1. Thus, provided that a molecule possesses at least one biological activity that is either similar to an activity of LFA-1 or inhibitory to such an activity, it is considered a "variant" of the alpha-subunit LFA-1, as that term is used herein, even if one of the molecules contains one or more amino acids not found in the other, or if the sequences of amino acid residues in the two molecules are not identical. Thus, for example, a compound lacking (or containing) one or more amino acid residues found (or not found) in the alpha-subunit of LFA-1 would be considered to be a variant of the alpha-subunit of LFA-1 if that compound possessed a biological activity similar to (or inhibitory to) a biological activity of the alpha-subunit of LFA-1. The term "biological activity" is intended to encompass "catalytic" as well as "structural" activity (i.e., the capacity to bind to another molecule, such as ICAM-1, another natural ligand of the LFA-1 molecule, the beta-subunit of LFA-1, or anti-alpha-subunit LFA-1 antibody), etc.

The present invention provides a method for producing functional derivatives of the alpha-subunit of the LFA-1 molecule. To obtain such derivatives, it is necessary only to mutagenize a DNA, RNA, or (more preferably) the cDNA sequence which encodes the LFA-1 alpha-subunit. Mutagenesis can either be random, or site specific. Further, mutagenesis may either be spontaneous or induced using chemical, radioactive, or recombinant techniques.

The scope of the present invention is further intended to include functional derivatives which lack certain amino acid residues, or which contain altered amino acid residues, so long as such derivatives exhibit the capacity to enhance or inhibit cellular adhesion.

Chemical mutagens include base analogs (such as, for example, 5-bromouracil, or 2-aminopurine); deaminating agents (such as, for example, nitrous acid, hydroxylamine, etc.); alkylating agents (such as, for example, methyl methanesulphonate, nitrosoguanidine, etc.); or intercolating agents (such as, for example, acridine orange, ethidium bromide, psoralen, etc.). Radiation-induced mutation can be caused by agents such as ultraviolet light, gamma, X ray, etc. Techniques for mutagenizing nucleic acid molecules may be found in Miller, J.H. (In: Experiments in Molecular Biology, Cold Spring Harbor Laboratory, Cold Spring Harbor, NY (1972)), and Silhavy, T.J., et al. (In: Experiments with Gene Fusions, Cold Spring Harbor Laboratory, Cold Spring Harbor, NY (1984)).

Site-specific mutagenesis may be employed to produce specific mutations as desired sites of the nucleic acid encoding the LFA-1 alpha-subunit. In brief, such procedures generally entail the synthesis of a synthetic oligonucleotide having a desired and defined DNA sequence. Methods for synthesizing such oligonucleotides are disclosed by Itakura, K., et al. (Ann. Rev. Biochem. 53:323-356 (1984)). A nucleic acid molecule which encodes the LFA-1 alpha-subunit protein, or a functional derivative thereof, is generally subcloned onto a double-stranded vector such as M13, φX174, etc., whose single strands may be separated one from another. A single strand of the vector is then incubated in the presence of the synthetic oligonucleotide. Since the DNA of the oligonucleotide is controllably defined, it is possible to construct an oligonucleotide capable of base pairing with any region of the LFA-1 alpha-subunit-encoding nucleic acid. Once base pairing has occurred between the oligonucleotide and the single-stranded plasmid, it is possible to extend the oligonucleotide using DNA polymerase to create a double-stranded DNA molecule which may then be sealed by DNA ligase. When this double stranded DNA molecule is introduced into a cell, semi-conservative DNA replication will result in the production of progeny molecules in which the DNA sequence of the oligonucleotide fragment has been incorporated into the LFA-1 alpha-subunit-encoding sequences.

The LFA-1 alpha subunit of the present invention, or its functional derivatives, may alternatively be prepared by synthetic chemical method using the well-known Merriefield or other techniques of peptide synthesis. Alternatively, such molecules may also be prepared by chemical synthesis of nucleic acid molecules (using, for example, phosphodiester synthesis techniques), which, upon expression, will result in their production.

Thus, if one desires to introduce a point mutation, and exogenous DNA sequence into a specific site in the LFA-1-encoding sequence, or to create a deletion of nucleotides normally present in such a sequence, one would design an oligonucleotide fragment which contains (or lacks) the mutation or sequence, and then pursue the above-described procedure. In order to introduce such a mutation or exogenous DNA sequence into a particular region of the LFA-1 alpha-subunit-encoding nucleic acid, it is necessary to surround the mutation or the exogenous DNA sequence with flanking DNA sequences that are complementary to the DNA sequence of the region whose mutagenesis is desired. (Jenkins, F., et al., Bioessays 5:244-247 (1986); Doerfler, W., Angew, Chem. Int. Ed. Engl. 23:919-931 (1984); Kaina, B., Biol. Zentralbl. 99:513-531 (1980); Kunkel, Proc. Natl. Acad. Sci. (USA) 82:488-492 (1985); Nisbet, I.T., et al., Gene Anal. Tech. 2:23-29 (1985); Hines, J.C., et al., Gene 11:207-218 (1980); Messing, J., et al., Nucl. Acid. Res. 9:309 (1981)).

Mutations can also be produced through the application of recombinant DNA technology. For example, the nucleotide sequence of a nucleic acid molecule which encodes the LFA-1 alpha-subunit can be scanned to identify oligonucleotide sites which are recognizable by restriction endonucleases. Such endonucleases can then be used to specifically cleave the nucleic acid sequence at a recognized site. By using a restriction endonuclease that recognizes (and cleaves at) two positions in the LFA-1-encoding sequence, it is possible to excise a fragment of the LFA-1 alpha-subunit-encoding sequence. Alternatively, it is possible to use two different endonucleases for this purpose. By incubating the cleaved molecules in the presence of DNA ligase, it is possible to reseal the LFA-1 alpha-subunit-encoding sequences to form a single sequence (which lacks the excised fragment). If no appropriate restriction endonuclease recognition sites exist in the LFA-1 alpha-subunit-encoding sequences, then such sites may be introduced into the sequences by the site-specific mutagenesis procedure described above.

Mutations may alternatively be introduced by cleaving the LFA-1 alpha-subunit-encoding sequence and "nibbling" the free termini with an exonuclease. By such treatment it is possible to introduce not only deletions, but frame-shift and other types of mutations. This technique is, moreover, capable of introducing novel restriction endonuclease sites into the LFA-1 alpha-subunit-encoding sequence. Methods for using restriction endonucleases, DNA ligases, and exonucleases are disclosed, for example, by Maniatis, T., et al. (In: Molecular Cloning, A Laboratory Manual, Cold Spring Harbor Laboratory, Cold Spring Harbor, NY (1982)).

Recombinant DNA techniques may also be used to produce fusion proteins composed of the LFA-1 alpha-subunit protein (or a functional derivative thereof) and a novel polypeptide. This novel polypeptide is not limited to any particular polypeptide and may comprise either a single amino acid or any set or permutation of amino acids. Such fusion molecules may be produced by ligating a DNA sequence which encodes the novel polypeptide to a DNA sequence which encodes the LFA-1 alpha-subunit (or functional derivative thereof), in a manner which does not introduce a frame-shift mutation. Examples of preferred polypeptides which may be fused to the LFA-1 alpha-subunit gene (or a functional derivative thereof) include eukaryotic or prokaryotic signal sequences (Gilbert, W., et al., U.S. Patent No. 4,411,994; Casadaban, M., et al., Proc. Natl. Acad. Sci. (USA) 76:4530-4533 (1979)), or polypeptides which extend (or diminish) the stability, biological half-life, or potency of the LFA-1 alpha-subunit (or a functional derivative thereof). An excellent review of the methodology of gene fusions is provided by Silhavy, T.J. et al. (In: Experiments with Gene Fusions, Cold Spring Harbor Laboratory, Cold Spring Harbor, NY (1984)).

Antibodies (especially monoclonal antibodies) may be elicited in response to immunization with fragments of the LFA-1 alpha-subunit. Such antibodies can be used to prevent the binding of some leukocytes to endothelial cells, antigen presenting cells or target cells and thus may be employed as anti-inflammatory agents.

Using the methods described above, fragments of the LFA-1 alpha-subunit may be prepared and assayed to determine whether they are antagonists of cellular adhesion. Fragments found to be antagonists of cellular adhesion may be employed as anti-inflammatory agents in accordance with the present invention.

The present invention derives in part from the discovery that leukocyte-substrate adhesion and leukocyte-endothelial cell adherence results from interactions involving the LFA-1 receptor. Since cellular adhesion is required in order that leukocytes may migrate to sites of inflammation and/or carry out various effector functions contributing to inflammation, agents which inhibit such cellular adhesion will attenuate or prevent inflammation. The LFA-1 receptor molecule is present on the surface of leukocyte cells. The adhesion of such cells to monolayers of endothelial cells, is mediated in part by LFA-1.

The interaction of LFA-1 molecules with their natural ligands (such as ICAM-1, etc.) is of central importance in cellular adhesion. Through the process of adhesion, lymphocytes are capable of continually monitoring an animal for the presence of foreign antigens. Although these processes are normally desirable, they are also the cause of organ transplant rejection, tissue graft rejection and many autoimmune diseases. Hence, any means capable of attenuating or inhibiting cellular adhesion would be highly desirable in recipients of organ transplants, tissue grafts or autoimmune patients.

Agents capable of antagonizing these interactions are highly suitable as anti-inflammatory agents in a mammalian subject. Significantly, such agents differ from general anti-inflammatory agents in that they are capable of selectively inhibiting adhesion, and do not offer other side effects such as nephrotoxicity which are found with conventional agents. Agents capable of binding to the alpha subunit of LFA-1, or to ICAM-1, or another natural ligand of the LFA-1 molecule can therefore be used to prevent organ or tissue rejection, graft versus host disease (i.e. a rejection of host tissue caused by a graft of bone marrow or other lymphocyte containing or generating tissue) or modify autoimmune responses by interfering with cell-bound LFA-1 without the fear of side effects (such as interaction with its cell-bound ligand, etc.).

Importantly, the use of agents capable of recognizing such molecules may permit one to perform organ transplants even between individuals having HLA mismatch.

Agents which interfere with the capacity of the LFA-1 receptor molecule to bind to its natural binding ligand are thus capable of impairing all of the above-decribed LFA-1-dependent functions. Hence, these agents may serve as anti-inflammatory agents in accordance with the present invention. Such agents include the LFA-1 alpha-subunit, LFA-1 (alpha and beta-subunits), and antibody capable of binding to the LFA-1 alpha-subunit, or to fragments of that subunit. All of such agents may be used in accordance with the present invention. The anti-inflammatory agents of the present invention are capable of treating inflammatory caused by a reaction of the specific defense system.

As used herein, the term "specific defense system" is intended to refer to that component of the immune system that reacts to the presence of specific antigens. Such cells include lymphocytes and macrophages. Inflammation is said to result from a response of the specific defense system if the inflammation is caused by, mediated by, or associated with a reaction of the specific defense system. Examples of inflammation resulting from a response of the specific defense system include the response to antigens such as rubella virus, autoimmune diseases, delayed-type hypersensitivity response mediated by T-cells (as seen, for example, in individuals who test "positive" in the Mantaux test), organ or tissue rejection, graft versus host disease (i.e. a rejection of host tissue caused by a graft of bone marrow or other lymphocyte containing or generating tissue), etc. The ability of LFA-1 alpha-subunit and its functional derivatives to antagonize such inflammatory reactions provides the basis for their therapeutic use in the treatment of chronic inflammatory diseases and autoimmune diseases such as lupus erythemotosus, autoimmune thyroiditis, experimental allergic encephalomyelitis (EAE), multiple sclerosis, some forms of diabetes, Reynaud's syndrome, rheumatoid arthritis, etc.

Since LFA-1 is expressed on cells which are capable of binding to endothelial tissue, the administration of the LFA-1 alpha-subunit, or LFA-1 (alpha and beta-subunits) to a patient provides a means for imaging or visualizing endothelial tissue. Moreover, this procedure provides diagnostic information concerning the quantity and distribution of the binding ligands of the LFA-1 receptor molecule which are present on the visualized tissue. In such a use, the LFA-1 alpha-subunits (or LFA-1 alpha beta receptor molecules) are detectably labeled, through the use of radioisotopes, affinity labels (such as biotin, avidin, etc.) fluoroescent labels, paramagnetic atoms, etc. Procedures for accomplishing such labeling are well known to the art. The antibodies (or fragments thereof) may be detectably labeled through the use of radioisotopes, enzyme labels, fluorescent labels, paramagnetic labels, electron-dense labels, toxin labels, etc. Preferred toxin labels include the diphtheria toxin, ricin, and cholera toxins. The administration of such labeled molecules into an individual will identify sites of inflammation. Such detectable labels can also be used to assay the status of a patient's immune system. Clinical application of antibodies in diagnostic imaging are reviewed by Grossman, H.B., Urol. Clin. North Amer. 13:465-474 (1986)), Unger, E.C. et al., Invest. Radiol. 20:963-700 (1985)), and Khaw, B.A. et al., Science 209:295-297 (1980)).

The ability of leukocytes to migrate spontaneously to sites of inflammation is dependent upon LFA-1 (Keizer, G.D., et al., Eur. J. Immunol. 17:1317-1322 (1987)). Such migration may be inhibited by administrating LFA-1 alpha-subunits, or LFA-1 (alpha and beta-subunit) to a patient. Similarly, the ability of leukocytes to adhere to endothelial cells has been found to be dependent upon LFA-1 Any of the anti-inflammatory agents of the present invention may be employed to inhibit such activities.

ICAMs (such as ICAM-1) are recognized by certain human viruses (particularly rhinoviruses of the major type (which bind to ICAM-1). These viruses bind to human cells by virtue of this recognition, and thereby mediate viral infection. Thus, a central step in the etiology of viral disease is the interaction between these cellular receptors and the virus.

Agents which suppress, compete with, or inhibit the ability of a virus to bind to an ICAM molecule thus have use in the treatment of viral (and particularly rhinoviral) infection.

One aspect of the present invention thus concerns the ability of the alpha-subunit of LFA-1 and its functional derivatives to interact with ICAM-1 and to thereby either prevent cell-viral attachment and viral infection, or to attenuate or diminish the severity or duration of such infection.

Of particular interest to the present invention are functional derivatives of the alpha-subunit of LFA-1 such as solubilized forms of the alpha-subunit of LFA-1, fragments of the alpha-subunit of LFA-1, etc. Such agents are preferably provided to a recipient patient as a heterodimer containing the molecule in association with a molecule of the beta-subunit of the CD-18 family. The above-described goal of treating viral infection may be accomplished with a single agent or with a combination of more than one agents.

For the purpose of treating viral infection, the above-described agent(s) of the present invention is to be provided to a recipient patient (for example, by intranasal means) at a dosage sufficient to permit the agent(s) to suppress, compete with, or inhibit the ability of a virus to bind to an ICAM molecule. Such a dosage shall, in general, be (for each agent provided) from 0.01 pg/kg patient weight to 1 mg/kg patient weight, although greater or less amounts can be employed.

For the purpose of treating viral infection, the administration of such agent(s) may be provided either "prophylactically" or "therapeutically." When provided prophylactically, the agent(s) are provided in advance of (i.e. prior to, at, or shortly after) the time of infection but in advance of any symptoms of viral infection. The prophylactic administration of the agent(s) serves to prevent or attenuate any subsequent infection. When provided therapeutically, the agent(s) are provided at (or shortly after) the onset of a symptom of actual viral infection (such as, for example, the appearance of virally induced nasal congestion, etc. or the detection of virus in bodily fluids, or the detection of antibodies, directed against the virus, in the serum of an infected patient, etc). The therapeutic administration of the agent(s) serves to attenuate any actual infection, and thus lessen its severity or duration.

## V. Administration of the LFA-1 Alpha-Subunit

The therapeutic effects of LFA-1 alpha-subunit may be obtained by providing to a patient the LFA-1 receptor molecule ($\alpha$- and $\beta$-subunits), the entire LFA-1 alpha-subunit molecule, or any therapeutically active functional derivative thereof. These molecules may be obtained either synthetically, or through the use of recombinant DNA technology, or from natural sources. Fragments of the LFA-1 receptor or its alpha-subunit may be additionally be obtained by proteolysis. The therapeutic advantages of these molecules may be augmented through the use of functional derivatives which possess additional amino acid residues added to enhance coupling to carrier or to enhance activity.

The molecules of the present invention are said to be "substantially free of natural contaminants" if preparations which contain them are substantially free of materials with which these products are normally and naturally found.

In providing a patient with the therapeutic molecules of the present invention, the dosage of administered agent will vary depending upon such factors as the patient's age, weight, height, sex, general medical condition, previous medical history, etc. In general, it is desirable to provide the recipient with a dosage of LFA-1 alpha-subunit (or a functional derivative thereof) which is in the range of from about 1 pg/kg to 10 mg/kg (body weight of patient), although a lower or higher dosage may be administered.

The molecules of the present invention may be administered to patients intravenously, intramuscularly, subcutaneously, enterally, or parenterally. Administration may be by continuous infusion, or by single or multiple boluses.

The anti-inflammatory agents of the present invention are intended to be provided to recipient subjects in an amount sufficient to suppress inflammation. An amount is said to be sufficient to suppress inflammation if the dosage, route of administration, etc. of the agent are sufficient to attenuate or prevent inflammation. The anti-inflammatory agents of the present invention may be provided either to provide to the onset of inflammation (so as to suppress the anticipated inflammation) or after the initiation or inflammation.

A composition is said to be "pharmacologically acceptable" if its administration can be tolerated by a recipient patient. Such an agent is said to be administered in a "therapeutically effective amount" if the amount administered is physiologically significant. An agent is physiologically significant if its presence results in a detectable change in the physiology of a recipient patient. The molecules of the present invention can be formulated according to known methods to prepare pharmaceutically acceptable compositions, whereby these materials, or their functional derivatives, are combined in admixture with a pharmaceutically acceptable carrier vehicle. Suitable vehicles and their formulation, inclusive of other human proteins, e.g., human serum albumin, are described, for example, in Remington's Pharmaceutical Sciences (16th ed.), Osol, A., ed., Mack, Easton, PA (1980). In order to form a pharmaceutically effective composition suitable for effective administration, such compositions will contain a therapeutically effective amount of LFA-1 alpha-subunit, or its fragments or functional derivatives, together with a suitable amount of carrier vehicle.

Additional pharmaceutical methods may be employed to control the duration of action. Controlled release preparations may be achieved through the use of polymers to complex or absorb LFA-1 alpha-subunit or its fragments or functional derivatives. The controlled delivery may be exercised by selecting appropriate macromolecules (for example, polyesters, polyamino acids, polyvinyl, pyrrolidone, ethylene vinylacetate, methylcellulose, carboxymethylcellulose, or protamine sulfate) and the concentration of macromolecules as well as the methods of incorporation in order to control release. Another possible method to control the duration of action by controlled release preparations is to incorporate LFA-1 alpha-subunit molecules, their fragments, or their functional derivatives, into particles of a polymeric material such as polyesters, polyamino acids, hydrogels, poly(lactic acid), or ethylene vinylacetate copolymers. Alternatively, instead of incorporating these agents into polymeric particles, it is possible to entrap these molecules in microcapsules prepared, for example, by coacervation techniques or by interfacial polymerization, for example, hydroxymethyl cellulose or gelatin-microcapsules and poly(methylmethacrylate) microcapsules, respectively, or in colloidal drug delivery systems, for example, liposomes, albumin microspheres, microemulsions, nanoparticles, and nanocapsules in macroemulsions. Such techniques are disclosed in Remington's Pharmaceutical Sciences (1980).

Having now generally described the invention, the same will be more readily understood through reference to the following examples which are provided by way of illustration, and are not intended to be limiting of the present invention, unless specified.

EXAMPLE 1

Purification of the LFA-1 Alpha-subunit

LFA-1 is expressed on the surfaces of SKW3 lymphocytes. The LFA-1 alpha-subunit molecule was purified from SKW3 cells by monoclonal antibody affinity chromatography using the method of Miller, L.J., et al. (J. Immunol. 137:2891-2900 (1987) which reference is incorporated by reference herein). The monoclonal antibody, TS1/22, which is directed against the alpha-subunit of LFA-1, was purified and coupled (using cyanogen bromide) to CL-4B Sepharose (Pharmacia) at 2 mg MAB per ml of packed bed. SKW3 cells (42.2 g), obtained from the MIT culture collection, were lysed in 300 ml of lysis buffer (Kurzinger, K. et al. J. Biol. Chem. 257:12412-12418 (1982)) and the lysate was then spun at 16,000 x g for 2 hours. The supernatant was then sequentially passed through a pre-column of activated and quenched CL-4B Sepharose and then a TS1/22 Sepharose column. The TS1/22 column was washed sequentially (Kurzinger, K. et al., J. Biol. Chem. 257:12412-12418 (1982)), and the LFA-1 alpha-subunit was eluted with 50 mM triethylamine, 0.5 M NaCl, 0.1% Triton X-100, 1 mM iodacetamide, 10 U/ml aprotinin, and 0.025% $NaN_3$, pH 11.5, and the pH immediately neutralized. The fractions containing LFA-1 alpha-subunit were pooled, lyophilized and precipated in 5 volumes of ethanol at -20°C overnight.

Purified protein was reduced and alkylated (Law, S.K.A. et al., EMBO J. 6:915-919 (1987)) and subjected to preparative SDS-polyacrylamide gel electrophoresis. The band corresponding to the LFA-1 alpha-subunit was visualized with 1 M KCl, excised and electroeluted (Pellegrino, M.A. et al., Clin. Immunol. Immunpath. 3:324-333 (1975)). The purified alpha-subunit was lyophilized and precipitated in 4 volumes of ethanol at -20°C overnight. The pellet was resuspended and digested with 1% trypsin (Wong, W.W. et al., Proc. Natl. Acad. Sci. (U.S.A.) 82:7711-7715 (1985)). The tryptic fragments were then isolated by HPLC (Beckman) on a C4 reverse phase column (Vydac). The peptides were eluted on a 0 to 60% acetonitrile gradient in 1% trifluoroacetic acid. Several peaks were rechromatographed isocratically in a concentration of acetonitrile determined by the equation:

$$F = (0.9E) - 2$$

where F is the volume percentage of acetonitrile under isocratic conditions for a peptide that eluted at E percent during the linear gradient (Lathe, R. et al. (J. Molec. Biol. 183:1-12 (1985)). Peaks were collected in 1.5 ml polypropylene tubes and concentrated to less than 50 μl. Eight peaks were subjected to microsequencing. The sequence of one peptide, L64, was used to synthesize a single oligonucleotide according to the suggestions of Lathe, R. et al. (J. Molec. Biol. 183:1-12 (1985)):
5′ - GGGATGTTGTGGTCATGGATGGTGGGCTCAAT - 3′

In summary, LFA-1 was initially isolated from SKW3, a T lymphoma cell line, lysate by monoclonal antibody affinity chromatography using an antibody directed against the α-subunit. SDS-PAGE fractions showed the α- and β-subunits. The α-subunit was further purified by preparative SDS-PAGE and the purified α-subunit was digested with trypsin and the peptides were isolated by reverse phase HPLC. The peptide sequence of nine peaks was determined by microsequencing (Figure 1). The peptide sequence that possessed the lowest codon redundancy was used to specify a single oligonucleotide sequence which utilized the most commonly occurring human codons (Lathe, R. et al., J. Molec. Biol. 183:1-12 (1985)). The sequences of the tryptic peptides of the LFA-1 alpha-subunit are shown in Table I.

TABLE I
SEQUENCES OF THE TRYPTIC PEPTIDES OF LFA-1 Alpha-subunit

| Residues | Amino Acid Sequence |
|---|---|
| 95-107 | X X D Q (N) T Y L S G L (E) Y L F |
| 199-210 | H M L L L T N T F G A I |
| 254-260 | Y I I G I G K |
| 405-413 | V L L F Q E X Q G |
| 494-503 | G E A I T A L T X I |
| 541-554 | I E G T Q V L S G I Q X F G |
| 564-576 | X (L) E (G) D (V/G) L A D V A V G A E |
| 803-817 | K V E M L K P H S E I X V S (T) |
| 929-946 | I (E/Q) P S I H N I P X L E A V X G |

Parentheses indicate ambiguity in sequence. The underlined residues were used to generate an oligonucleotide probe.

EXAMPLE 2

cDNA Cloning

Recombinants ($5 \times 10^5$) from a λgt 10 library selected for cDNA inserts greater than 2 kb and produced from PMA (Phorbol Myristic Acid)-induced HL-60 cells (Corbi, A. et al., EMBO J. 6:4023-4028 (1987)) were plated at a density of 50,000 recombinants per 150 mm plate. The library was amplified (Woo, S.L.C., Met. Enzymol. 68:389-395 (1979)) and nitrocellulose filters were processed according to the guidelines of Benton and Davis (Benton, W.D. et al., Science 196: 180-182 (1977)). The filters were prehybridized in 6 x SSC [0.6 M NaCl, 0.06 M Sodium Citrate], 0.05% sodium phosphate and sodium pyrophosphate, 0.5% SDS, 1 x Denhardt's and 100 μg/ml of salmon sperm DNA overnight at 42°C. The single sequence oligonucleotide (sequence shown above) was labeled with $\gamma^{32p}$-ATP using polynucleotide kinase. The filters were hybridized overnight in the prehybridization buffer at 42°C and then washed sequentially in 6 x SSC, 0.05% sodium pyrophosphate for 15 min. at 50°C. Filters were exposed on preflashed XAR-5 film from 6 to 20 hours. Phage that gave signals on duplicate filters were plaque purified, and their cDNA inserts were sized by electrophoresis on agarose gels.

The 32-mer oligonucleotide probe was used to isolate twenty clones from a size-selected λgt10 cDNA library constructed from PMA-stimulated myeloid cells (Corbi, A. et al., EMBO J. 6:4023-4028 (1987)). These cells have been previously shown to synthesize the LFA-1 α-subunit (Miller, L.J. et al., J. Immunol. 139:842-847 (1987)). The insert size was determined and the longest clone, λ5L5, was restriction mapped (Figure 2). This clone contained the nucleotide sequence corresponding to the oligonucleotide probe (85% identity to the "best-guess" probe). The nucleotide sequence also encoded the tryptic peptide that was determined by microsequencing (16 of 16 residues) which included and extended beyond the oligonucleotide probe. However, this clone did not encode the entire protein since not all of the tryptic peptide sequences were present in the open reading frame. The 5′ 1.0 kb EcoRI fragment of λ5L5 was used to rescreen another $5 \times 10^5$ recombinants. An additional 14 clones were identified, and the clone, λ3R1, had an identical restriction map in the overlapping regions and contained an additional 1.0 kb 5′ fragment. The nucleotide sequence of this additional fragment was determined (Figure 2).

## EXAMPLE 3

Restriction Mapping and Nucleotide Sequencing

Restriction maps of the selected clones were determined by double and partial digests (Maniatis, T., et al. (In: Molecular Cloning, A Laboratory Manual, Cold Spring Harbor Laboratory, Cold Spring Harbor, NY (1982))). Restriction fragments were subcloned into either M13mp18 and M13mp19 (Messing, J. Met. Enzymol. 101:20-78 (1983)) or pGEM-3Z, 4Z, or 7Z (Promega). Deletions of the fragments in pGEM were made using Exonuclease III and S1 (Henikoff, S., Gene 28:351-359 (1984)). Sequencing was by the dideoxy termination method (Sanger, F. et al., Proc. Natl. Acad. Sci. (U.S.A.) 74:5463-5467 (1977)). The coding sequence, 5′ and 3′ untranslated regions were determined 100%, 83.1% and 33.7% in both orientations, respectively.

The overlapping cDNA clones contain 5139 nucleotides (Figure 3). There is an open reading frame of 3510 nucleotides, a 5′ untranslated region of 94 nucleotides and a 3′ untranslated region of 1535 nucleotides which contains a polyadenylation site 15 nucleotides before the poly(A+) tail. Within the 3′ untranslated region there is a typical Alu1 repeat consisting of two tandem related sequences each terminated by an A-rich segment (Hardman, N., Biochem. J. 234:1-11 (1986)). The Alu sequence is 304 nucleotides long, and is 78.8% identical to the common Alu family sequence.

## EXAMPLE 4

Southern and Northern Blot

The Southern blot was performed as described by Corbi, A. et al. (J. Exper. Med. 167:1597-1607 (1988)). 20 μg/ml of total cellular RNA isolated from either SKW3, U937, IB4 or EJ cells was electrophoresed on a 1.0% formaldehyde gel and transferred to nitrocellulose (In: Current Protocols in Molecular Biology, Green Publishing Associates and Wiley-Interscience, New York). The nylon membrane (Zeta probe (Biorad)) was prehybridized and hybridized in 2 x SSC, 1 x Denhardt's solution, 0.1% SDS and 10 μg/ml of herring sperm DNA. A 1.8 kb Eco RI probe from the 5′ end of the cDNA clone. λ3R1, was labeled by nick translation and used as probe.

Northern blot analysis demonstrated a message of 5.5 kb in SKW3, U937, and IB4 cells but a signal was not detected in EJ cells. The cell distributions are in good agreement with the size of the cDNA clone and the cell surface expression of LFA-1. Southern blot analysis using the 1.2kb 5′ EcoRI-BamHI fragment from clone λ2L2 hybridized to two fragments of 10 and 8 kb (Corbi, A. et al.,, J. Exper. Med. 167:1597-1607 (1988)). A genomic clone isolated from a cosmid library posseses these two fragments of identical length. These fragments are contiguous and demonstrate that LFA-1 is a single copy gene.

## EXAMPLE 5

Computer Analysis

Homology searches and alignment of sequences initially used the Microgenie DNA program (Beckman), FASTP (Wilbur and Sigmann) on the NBRF and NEW databases (National Biomedical Research Foundation, Washington, DC), and FASTP using the SWISS-PROT data base (Bionet). These alignments were then optimized using ALIGN (NBRF) (5585) and GENALIGN (Bionet).

Hydrophobicity was determined using Microgenie DNA program as well as PEP-PLOT (5792) (University of Wisconsin Genetics Computer Group). Hydrophobicity analysis of the amino acid sequence showed that LFA-1 $\alpha$-subunit is a typical transmembrane protein with a hydrophobicity signal sequence, an extracellular domain, a single hydrophobic transmembrane region, and a short cytoplasmic tail. The N-terminal residue of human LFA-1 was identified by homology to the N-terminal of murine LFA-1 (Figure 5). Human LFA-1 has 55% identity with murine LFA-1 over the first 20 amino acids. A classical signal peptide with a consensus sequence (Ala-X-Ser/Pro) for the cleavage peptidase precedes the N-terminal sequence. Thre are three putative upstream transcription initiation sites (ATG) in frame. The use of the first initiation site at nucleotide position 89 is generally favored (Kozak, M. Nucl. Acid Res. 12:857-872 (1984)) and gives a twenty-five residue signal sequence with several polar groups near the N-terminal residues which is typically found in signal sequences (von Heijne, G., J. Molec. Biol. 173:243-251 (1984)). All 117 amino acids determined by microsequencing of tryptic peptides were found in the translated open reading frame, confirming the authenticity of the cDNA clones. Taken together, these findings demonstrate that the LFA-1 $\alpha$-subunit has an N-terminal 29 + amino acid signal sequence, an extracellular domain of 1063 residues, a transmembrane domain of 29 amino acids, and a C-terminal cytoplasmic tail of 53 amino acids.

Seven repeats of 49-63 amino acids are within the extracellular domain. The degree of internal identity and statistical significance among these internal repeats is highest among the last three repeats, 24-33% with $p<10^{-2}$ to $<10^{-6}$. The central region of the latter three repeats has homology with several divalent cation binding sites (Figure 4). Since previous studies have shown that $Mg^{2+}$ along or at lower concentration in conjunction with $Ca^{2+}$ is necessary for functional integrity of the protein (Rothlein, R. et al., J. Exper. Med. 163:1132-1149 (1986)). Homology of the three tandem repeats with divalent cation binding sites suggests that LFA-1 binds divalent cations. Repeats I-IV lack the consensus divalent cation binding site but have conserved flanking regions. These structural similarities raise the possibility that these repeats arose by a duplication event.

The mature protein has a Mr = 126,193 and twelve N-linked glycosylation sites (Asn-X-Thr/Ser) are located in the extracellular domain. Previous studies have demonstrated that at least five of these sites are glycosylated and have oligosaccharides with a $M_r$ = 5,000-10,000 daltons which is typical for complex N-linked carbohydrate (Dahms, N.M. et al., J. Immunol. 134:3978-3986 (1985)). Therefore, the use of 5-10 of these glycosylation sites would predict a molecular weight in agreement with the molecular weight as determined by SDS-PAGE.

The determination of the N-terminal of murine LFA-1 (Girma, J-P. et al., Blood 70:605-611 (1987)) and the primary structure of the LFA-1/Mac-1/ p150,95 common $\beta$-subunit has suggested that the LFA-1 $\alpha$-subunit is a member of the integrin superfamily. The LFA-1 $\alpha$-subunit has a striking homology (35% identity) to the p150,95 and Mac-1 $\alpha$-subunits and, to a lesser extent, to the ECM receptors $\alpha$-subunits (25% identity). On the other hand, the FNR, VNR, and IIb are 40% identical to each other. Additionally, the leukocyte integrins also contain an insertion of approximately 200 amino acids near the N-terminal region of the protein that is not present in the three sequenced ECM receptors (Figure 5). Furthermore, the region in which a protease cleavage site occurs in the VNR, FNR, and gpIIb/IIIa is absent from the LFA-1 as well as the p150,95 and Mac-1 $\alpha$ subnits and correlates with the lack of proteolytic processing of the LFA-1 family $\alpha$-subunits. As a whole, these structural features define two subfamilies of the $\alpha$-subunit integrins, the leukocyte integrins and the ECM integrins.

All the $\alpha$-subunits of the integrin family have tandem repeats similar to LFA-1 which contain putative divalent cation binding sites (Corbi, A. et al., EMBO J. 6:4023-4028 (1987), Poncz, M. et al., J. Biol. Chem. 262:8476-8482 (1987), Suzuki, S. et al., Proc. Natl. Acad. Sci (U.S.A. 83:8614-8618 (1986), Argaves, W.S. et al., J. Cell Biol. 105:1183-1190 (1987), Ruoslahti, E. et al., Science 238:491-497 (1987)). However, only three of the repeats in LFA-1 and p150 contain putative metal binding sites while all four of the repeats in the ECM receptors contain metal binding sites. The putative divalent cation binding sites of LFA-1 and the other integrins are related but not identical to previously described divalent cation binding sites of integral membrane proteins. Several types of divalent cation binding sites exist. The first set of sites have an alternating D-X-D(N)-X-D(N) structure. These sites are found in Troponin C, parvalbumin (5256), and galactose binding protein, among others. These sites form an EF loop structure and have 6-7 chelation sites. The putative binding sites on LFA-1 and the other integrins also have a D-X-D(N)-X-D(N) primary structure with a number of G residues between the chelating residues being conserved. However, the residue in the -Z position is replaced by a hydrophobic residue. Furthermore, the $\alpha$ helix before and after the calcium binding site appears to be absent from the integrin sites (Corbi, A. et al., EMBO J. 6:4023-4028 (1987)). The significance of this change is unknown, but may partially account for the preference of these sites for $Mg^{2+}$.

A search of the NBRF ad Swiss-Protein data banks revealed that this LFA-1 family specific domain, which we will refer to as the "L" domain, has significant homology with the A1 domain of human vWF, human complement factor B, and chicken collagen matrix protein. These similarities extend over the entire length of the L domain. Since vWF has three repeat A domains (A1, A2, A3) (5790), the domain in the complement factor B is analogous to a similar domain in complement component 2 (C2) (5789), and the CMP has 2 repeats (5778), the LFA-1 L domain was compared against all these domains using the ALIGN program. With the exception of C2, all these alignments were statistically significant (Figure 6).

These similarities implicate a possible functional domain for LFA-1. First, the A1 domain of vWF binds to glycoprotein Ib and heparin (5646) while both the A1 and A3 domains are involved in the binding to collagen. Second, the homologous domain in factor B is located on the C-terminal side of the cleavage site at which factor B is cleaved to factor Bb and on the N-terminal side of the serine protease (Mole, J.E. et al., J. Biol. Chem 259:3407-3412 (1984), Bently, D.R., Biochem. J. 239:339-345 (1986)). This domain is, therefore, located in the region which would be expected to interact with its ligand, C3b. The structure of collagen matrix protein has been partially determined and has two repeats separated by an epidermal growth factor-like sequence (Argaves, W.S. et al., Proc. Natl. Acad. Sci. (U.S.A.) 84:464-468 (1987)). CMP interacts with both collagen (Argaves, W.S. et al., Proc. Natl. Acad. Sci. (U.S.A.) 84:464-468 (1987)) and cartilage proteoglycan (Figure 7) (Paulsson, M. et al. Collagen Rel. Res. 4:219-229 (1984)).

LFA-1, Mac-1, and p150,95 have been recently localized to chromosome l6p11 (Corbi, A. et al.,, J. Exper. Med. 167:1597-1607 (1988)). The structural similarity and the proximity of the genes encoding the leukocyte integrin α-subunits suggests that a primordial gene duplicated and gave rise to at least two branches of integrin α-subunits, the leukocyte integrins and the ECM integrins. A 180 amino acid domain became inserted in the primordial gene of the leukocyte integrin α-subunit and then the gene duplicated and gave rise to LFA-1, Mac-1, and p150,95. The similarity of these domains to the LFA-1 family L domain suggests that this L domain may be a functional domain. Furthermore, these homologies imply that a primordial domain became inserted into several proteins and then evolved diverse recognition functions in hemostasis, complement activation, and the immune response.

These structural differences between the two groups of α-subunit integrins correlate with the difference in the recognition site of the function domains since RGD-containing peptides will block the binding of the FNR and gpIIb/IIIa to their respective ligands (Ruoslahti, E. et al., Science 238:491-497 (1987)) while an RGDS peptide will not inhibit LFA-1 interaction with ICAM-1. Unlike the ligands of the ECM integrins, ICAM-1 itself does not have an RGD peptide sequence and is a member of the immunoglobulin gene superfamily. Both structural and functional data suggest that the integrin superfamily can be divided into two subfamilies of α-subunits. These structural and functional properties of the α-subunits associated with $\beta_2$, however, may not be unique to their subfamily since several other integrin α-subunits may have structural characteristics similar to the LFA-1 family such as a lack of a proteolytic cleavage site (Charo, I.F. et al., Proc. Natl. Acad. Sci. (U.S.A.) 83:8351-8355(1986)).

The elucidation of the structure of the LFA-1 α-subunit revealed novel evolutionary relationships among the integrin α-subunits and suggested a possible functional domain, and demonstrated that the LFA-1 α-subunit belongs to the integrin superfamily but possesses an additional domain. This L domain and homologous domains constitute a protein "domain" family that is of functional importance. Since the recognition site of LFA-1 is not RGD, the L domain may be of functional significance in the LFA-1 α-subunit as well as the other leukocyte integrins, Mac-1 and p150,95. However, since LFA-1 is involved in a large number of leukocyte functions and may have more than one ligand (Rothlein, R., et al., J. Immunol. 137:1270-1274 (1986)), it is possible that more than one functional domain exists in the LFA-1 α-subunit. It is also of interest to dissect the interactions the cytoplasmic tail will have with the cytoskeleton and what role this interaction plays in signalling. The availability of cDNA clones for the α- and β-subunits allow these structure-function relationships to be examined.

## Claims

1. Human LFA-1 alpha-subunit having an amino acid sequence according to Figure 3, or a functional derivative thereof substantially free of natural contaminants.

2. The human LFA-1 alpha-subunit or functional derivative thereof of claim 1, wherein said molecule is additionally capable of binding to ICAM-1 or another natural ligand of LFA-1.

3. The human LFA-1 alpha-subunit or functional derivative thereof of claim 1, wherein said molecule is capable of associating with an LFA-1 beta subunit to form an LFA-1 heterodimer.

4. The human LFA-1 alpha-subunit or functional derivative thereof of claim 3, wherein said LFA-1 heterodimer is capable of binding to ICAM-1 or another natural ligand of LFA-1.

5. The human LFA-1 alpha-subunit or functional derivative thereof of claim 1, wherein said molecule contains at least one polypeptide selected from the group consisting of:

| | |
|---|---|
| a. P-P-R-A-G-R-H; | h. G-V-D-V-Q-D-G-E-I-E; |
| b. I-I-T-D-G-E-A; | i. D-I-N-G-D-G-L-V-D-V; |
| c. D-W-A-G-G-F-L; | j. V-K-D-L-E-G-D-G-L-A; |
| d. S-Q-V-Q-T-I-H; | k. T-Y-L-S-G-L; |
| e. R-H-G-G-L-S-P; | l. Y-I-I-G-I-G-K; |
| f M-S-C-T-D-F-S; | m. I-E-G-T-Q-V-L-S-Q; and |
| g. R-L-L-S-R-A-L; | n. P-S-I-H-N-I-P. |

6. The functional derivative of the human LFA-1 alpha-subunit of any one of claims 1 to 5, wherein said functional derivative is a fragment of the LFA-1 alpha-subunit.

7. The functional derivative of the human LFA-1 alpha-subunit of claim 1 wherein said functional derivative is a chemical derivative of LFA-1 alpha-subunit.

8. A recombinant DNA molecule coding for a human LFA-1 alpha-subunit or a functional derivative thereof according to any one of claims 1 to 7.

9. The DNA molecule of claim 8, wherein said human LFA-1 alpha-subunit or said functional derivative thereof contains at least one polypeptide selected from the group consisting of:

| | |
|---|---|
| a. P-P-R-A-G-R-H; | h. G-V-D-V-Q-D-G-E-I-E-; |
| b. I-I-T-D-G-E-A; | i. D-I-N-G-D-G-L-V-D-V; |
| c. D-W-A-G-G-F-L; | j. V-K-D-L-E-G-D-G-L-A; |
| d. S-Q-V-Q-T-I-H; | k. T-Y-L-S-G-L; |
| e. R-H-G-G-L-S-P; | l. Y-I-I-G-I-G-K; |
| f. M-S-C-T-D-T-S; | m. I-E-G-T-Q-V-L-S-Q; and |
| g. R-L-L-S-R-A-L; | n. P-S-I-H-N-I-P. |

10. Human LFA-1 alpha-subunit or functional derivative thereof according to any one of claims 1 to 7 for the use in the suppression of inflammation, the suppression of metastasis of an hematopoietic tumor cell which requires a functional member of the LFA-1 family for migration, the suppression of growth of an LFA-1 alpha-subunit expressing tumor cell or the treatment of a viral infection.

11. A method of diagnosing the presence and location of inflammation resulting from a response of the specific defense system in a mammlian subject suspected of having said inflammation which comprises:

(a) incubating a sample of tissue of said subject with a composition containing a nucleic acid molecule capable of binding to a DNA according to claims 8 or 9, wherein said binding is capable of identifying a cell which is capable of binding ICAM-1, or another natural ligand of LFA-1, and

(b) detecting said nucleic acid molecule.

12. A method of diagnosing the presence and location of inflammation resulting from a response of the specific defense system in a mammalian subject of having said inflammation which comprises:

(a) incubating a sample of tissue of said subject with a composition containing a detectably labeled human LFA-1 alpha-subunit or functional derivative theeof according to any one of claims 1 to 7, capable of identifying a cell which expresses ICAM-1, or another natural ligand of human LFA-1, and

(b) detecting said human LFA-1 alpha-subunit.

**13.** A method of diagnosing the presence and location of a tumor cell which expresses ICAM-1 or another natural ligand of human LFA-1, in a subject suspected of having such a cell, wich comprises:

(a) incubating a sample of tissue of said subject in the presence of a composition containing a detectably labeled binding ligand capable of binding to said ICAM-1, or another natural ligand of human LFA-1, said binding ligand being LFA-1 alpha subunit or a functional derivative thereof according to any one of claims 1 to 7 and

(b) detecting said binding ligand which is bound to said ICAM-1 present in said sample of tissue.

**14.** Use of the alpha-subunit of human LFA-1 or a functional derivative thereof according to any one of claimes 1 to 7 for the manufacture of a pharmaceutical composition for the treatment of viral infections.

## Patentansprüche

**1.** Humane LFA-1 alpha-Untereinheit, mit einer Aminosäuresequenz gemäß Abbildung 3 oder ein funktionelles Derivat davon, im wesentlichen frei von natürlichen Verunreinigungen.

**2.** Humane LFA-1 alpha-Untereinheit oder ein funktionelles Derivat davon nach Anspruch 1, worin dieses Molekül zusätzlich in der Lage ist, an ICAM-1 oder einen anderen natürlichen Liganden von LFA-1 zu binden.

**3.** Humane LFA-1 alpha-Untereinheit oder ein funktionelles Derivat davon nach Anspruch 1, worin dieses Molekül in der Lage ist, mit einer LFA-1 beta-Untereinheit zu assoziieren, um ein LFA-1 Heterodimer zu bilden.

**4.** Humane LFA-1 alpha-Untereinheit oder ein funktionelles Derivat davon nach Anspruch 3, worin dieses LFA-1 Heterodimer in der Lage ist, an ICAM-1 oder einen anderen natürlichen Liganden von LFA-1 zu binden.

**5.** Humane LFA-1 alpha-Untereinheit oder ein funktionelles Derivat davon nach Anspruch 1, worin dieses Molekül wenigstens ein Polypeptid enthält, welches aus folgender Gruppe ausgewählt wird:

| | |
|---|---|
| a. P-P-R-A-G-R-H, | h. G-V-D-V-Q-D-G-E-I-E; |
| b. I-I-T-D-G-E-A, | i. D-I-N-G-D-G-L-V-D-V; |
| c. D-W-A-G-G-F-L; | j. V-K-D-L-E-G-D-G-L-A; |
| d. S-Q-V-Q-T-I-H; | k. T-Y-L-S-G-L; |
| e. R-H-G-G-L-S-P; | l. Y-I-I-G-I-G-K; |
| f. M-S-C-T-D-F-S; | m. I-E-G-T-Q-V-L-S-Q; and |
| g. R-L-L-S-R-A-L; | n. P-S-I-H-N-I-P. |

**6.** Funktionelles Derivat der humanen LFA-1 alpha-Untereinheit nach einem der Ansprüche 1 bis 5, worin dieses funktionelle Derivat ein Fragment der LFA-1 alpha-Untereinheit ist.

**7.** Funktionelles Derivat der humanen LFA-1 alpha-Untereinheit nach Anspruch 1, worin dieses funktionelle Derivat ein chemisches Derivat der LFA-1 alpha-Untereinheit darstellt.

**8.** Rekombinantes DNA Molekül, welches für eine humane LFA-1 alpha-Untereinheit oder ein funktionelles Derivat davon nach einem der Ansprüche 1 bis 7 kodiert.

**9.** DNA Molekül nach Anspruch 8, worin diese humane LFA-1 alpha-Untereinheit oder das funktionelle Derivat davon wenigstens ein Polypeptid enthält, welches aus folgender Gruppe ausgewählt wird:

| a. P-P-R-A-G-R-H; | h. G-V-D-V-Q-D-G-E-I-E-; |
|---|---|
| b. I-I-T-D-G-E-A; | i. D-I-N-G-D-G-L-V-D-V; |
| c. D-W-A-G-G-F-L; | J. V-K-D-L-E-G-D-G-L-A; |
| d. S-Q-V-Q-T-I-H; | k. T-Y-L-S-G-L; |
| e. R-H-G-G-L-S-P; | l. Y-I-I-G-I-G-K; |
| f. M-S-C-T-D-T-S; | m. I-E-G-T-Q-V-L-S-Q; and |
| g. R-L-L-S-R-A-L; | n. P-S-I-H-N-I-P. |

**10.** Humane LFA-1 alpha-Untereinheit oder ein funktionelles Derivat davon nach einem der Ansprüche 1 bis 7 zur Verwendung bei der Suppression von Entzündungen, der Suppression von Metastasen haematopoetischer Tumorzellen, die ein funktionelles Mitglied der LFA-1 Familie für Migration sein müssen, der Suppression des Wachstums von Tumorzellen, die LFA-1 alpha-Untereinheit exprimieren oder zur Behandlung einer viralen Infektion.

**11.** Verfahren zum Diagnostizieren einer Entzündung und ihres Ortes, die von der Antwort des spezifischen Abwehrsystems eines Säugers verursacht worden ist, der vermutlich diese Entzündung hat, das folgende Maßnahmen umfaßt:

(a) Inkubation einer Gewebeprobe des Individuums mit einem Mittel, das ein Nukleinsäuremolekül enthält, welches in der Lage ist, an eine DNA gemäß den Ansprüchen 8 oder 9 zu binden, wobei diese Bindung eine Zelle zu identifizieren vermag, die an ICAM-1 oder an einen anderen natürlichen Liganden von LFA-1 zu binden vermag, und

(b) Nachweis dieses Nukleinsäuremoleküls.

**12.** Verfahren zum Diagnostizieren einer Entzündung und ihres Ortes, die von der Antwort des spezifischen Abwehrsystems eines Säugers verursacht worden ist, der vermutlich diese Entzündung hat, das folgende Maßnahmen umfaßt:

(a) Inkubation einer Gewebeprobe des Individuums mit einem Mittel, das eine auffindbar markierte humane LFA-1 alpha-Untereinheit oder ein funktionelles Derivat davon gemäß einem der Ansprüche 1 bis 7 enthält, die bzw. das Zellen zu identifizieren vermag, die ICAM-1 oder einen anderen natürlichen Liganden von humanem LFA-1 exprimieren können, und

(b) Nachweis dieser humanen LFA-1 alpha-Untereinheit.

**13.** Verfahren zum Nachweis und zur Lokalisation einer Tumorzelle, die ICAM-1 oder einen natürlichen Liganden von humanem LFA-1 expremiert, bei einem Patienten, der vermutlich eine derartige Zelle hat, das folgende Maßnahmen umfaßt:

(a) Inkubation einer Gewebeprobe des Individuums mit einem Mittel, das einen auffindbar markierten Bindungs-Liganden enthält, der ICAM-1 oder einen anderen natürlichen Liganden von humanem LFA-1 binden kann, wobei dieser Bindungsligand die LFA-1 alpha-Untereinheit oder ein funktionales Derivat davon nach einem der Ansprüche 1 bis 7 ist, und

(b) Nachweis dieses Bindungsliganden, der in der Gewebeprobe an ICAM-1 gebunden ist.

**14.** Verwendung der alpha-Untereinheit von humanem LFA-1 oder eines funktionellen Derivates davon gemäß einem der Ansprüche 1 bis 7 zur Herstellung eines pharmazeutischen Mittels zur Behandlung viraler Infektionen.

**Revendications**

**1.** Sous-unité alpha de LFA-1 humain ayant une séquence d'aminoacides selon la figure 3, ou un de ses dérivés fonctionnels, essentiellement sans contaminants naturels.

**2.** Sous-unité alpha de LFA-1 humain ou un de ses dérivés fonctionnels selon la revendication 1, ladite molécule étant de plus capable de se lier à l'ICAM-1 ou à un autre ligand naturel de LFA-1.

**3.** Sous-unité alpha de LFA-1 humain ou un de ses dérivés fonctionnels selon la revendication 1, ladite molécule étant capable de s'associer avec une sous-unité bêta de LFA-1 pour former un hétérodimère de LFA-1.

4. Sous-unité alpha de LFA-1 humain ou un de ses dérivés fonctionnels selon la revendication 3, ledit hétérodimère de LFA-1 étant capable de se lier à l'ICAM-1 ou à un autre ligand naturel de LFA-1.

5. Sous-unité alpha de LFA-1 humain ou un de ses dérivés fonctionnels selon la revendication 1, ladite molécule contenant au moins un polypeptide choisi dans le groupe constitué par

| | |
|---|---|
| a. P-P-R-A-G-R-H; | h. G-V-D-V-Q-D-G-E-I-E; |
| b. I-I-T-D-G-E-A; | i. D-I-N-G-D-G-L-V-D-V; |
| c. D-W-A-G-G-F-L; | j. V-K-D-L-E-G-D-G-L-A; |
| d. S-Q-V-Q-T-I-H; | k. T-Y-L-S-G-L; |
| e. R-H-G-G-L-S-P; | l. Y-I-I-G-I-G-K; |
| f. M-S-C-T-D-F-S; | m. I-E-G-T-Q-V-L-S-Q; et |
| g. R-L-L-S-R-A-L; | n. P-S-I-H-N-I-P. |

6. Dérivé fonctionnel de la sous-unité alpha de LFA-1 humain selon l'une quelconque des revendications 1 à 5, ledit dérivé fonctionnel étant un fragment de la sous-unité alpha de LFA-1.

7. Dérivé fonctionnel de la sous-unité alpha de LFA-1 humain selon la revendication 1, ledit dérivé fonctionnel étant un dérivé chimique de la sous-unité alpha de LFA-1.

8. Molécule d'ADN recombiné codant pour une sous-unité alpha de LFA-1 humain ou un de ses dérivés fonctionnels selon l'une quelconque des revendications 1 à 7.

9. Molécule d'ADN selon la revendication 8, ladite sous-unité alpha de LFA-1 humain ou ledit dérivé fonctionnel contenant au moins un polypeptide choisi dans le groupe constitué par

| | |
|---|---|
| a. P-P-R-A-G-R-H; | h. G-V-D-V-Q-D-G-E-I-E; |
| b. I-I-T-D-G-E-A; | i. D-I-N-G-D-G-L-V-D-V; |
| c. D-W-A-G-G-F-L; | j. V-K-D-L-E-G-D-G-L-A; |
| d. S-Q-V-Q-T-I-H; | k. T-Y-L-S-G-L; |
| e. R-H-G-G-L-S-P; | l. Y-I-I-G-I-G-K; |
| f. M-S-C-T-D-T-S; | m. I-E-G-T-Q-V-L-S-Q; et |
| g. R-L-L-S-R-A-L; | n. P-S-I-H-N-I-P. |

10. Sous-unité alpha de LFA-1 humain ou un de ses dérivés fonctionnels selon l'une quelconque des revendications 1 à 7, pour l'utilisation dans la suppression de l'inflammation, la suppression de la métastase d'une cellule tumorale hématopoïétique qui nécessite pour la migration un membre fonctionnel de la famille des LFA-1, la suppression de la croissance d'une cellule tumorale exprimant une sous-unité alpha de LFA-1 ou le traitement d'une infection virale.

11. Procédé pour diagnostiquer la présence et la localisation d'une inflammation résultant d'une réponse du système de défense spécifique chez un sujet mammifère soupçonné d'avoir ladite inflammation, qui comprend
   (a) l'incubation d'un échantillon de tissu dudit sujet avec une composition contenant une molécule d'acide nucléique capable de se lier à un ADN selon les revendications 8 ou 9, cette liaison étant capable d'identifier une cellule susceptible de fixer l'ICAM-1 ou un autre ligand naturel de LFA-1, et
   (b) la détection de ladite molécule d'acide nucléique.

12. Procédé pour diagnostiquer la présence et la localisation d'une inflammation résultant d'une réponse du système de défense spécifique chez un sujet mammifère soupçonné d'avoir ladite inflammation, qui comprend
   (a) l'incubation d'un échantillon de tissu dudit sujet avec une composition contenant une sous-unité alpha de LFA-1 humain ou un de ses dérivés fonctionnels selon l'une quelconque des revendications 1 à 7, marqué(e) par un marqueur décelable, et capable d'identifier une cellule exprimant l'ICAM-1 ou un autre ligand naturel de LFA-1 humain, et

(b) la détection de ladite sous-unité alpha de LFA-1 humain.

**13.** Procédé pour diagniostiquer la présence et la localisation d'une cellule tumorale qui exprime l'ICAM-1 ou un autre ligand naturel de LFA-1 humain chez un sujet soupçonné d'avoir une telle cellule, qui comprend:

(a) l'incubation d'un échantillon de tissu dudit sujet en présence d'une composition contenant un ligand de liaison marqué par un marqueur décelable, capable de se lier à ladite ICAM-1 ou à un autre ligand naturel de LFA-1 humain, ledit ligand de liaison étant une sous-unité alpha de LFA-1 ou un de ses dérivés fonctionnels selon l'une quelconque des revendications 1 à 7, et

(b) la détection dudit ligand de liaison qui est lié à ladite ICAM-1 présente dans ledit échantillon de tissu.

**14.** Utilisation de la sous-unité alpha de LFA-1 humain ou d'un de ses dérivés fonctionnels selon l'une quelconque des revendications 1 à 7 pour la préparation d'une composition pharmaceutique pour le traitement d'infections virales.

Figure 1

24

1.0 kb

Bl P Sc P NC BPB P P R P S P Ba P PBa Sp

λ 3R1

λ 515

Figure 2

Figure 3.

Flanking consensus sequence (LFA-1):     Y F G Y S I - - - - - - - - - - - - - - - L A V G A P
                                            L        V                                 V

Flanking consensus sequence (integrins):  S Y F G - S V - - - - - - - - - - - - - - L V V G A P

I    7     G A R S F S P P R A G R I F G Y R V L Q V - - - - G N G - - - - V I V G A P G F - G N S T G S - L Y Q C Q S G T G H    54
II   57    P V T L R G S N Y T S K Y L G - - M T L A T D - P T D G - - S I L A C D - P G L - S R T C D Q N T Y L S G - L C Y L E    107
III  314   K Q D L T S F N M E L S S S G I S A D L S - - - - - R G H A V V G A V G A K D W A - - - - G G F L D L K A D L Q D D T F I G    366
IV   367   N E P L T P - E V R A G Y L G Y T V T W I P S R Q - - - K T S L L A S G A P R - Y Q H M G R V L L F Q E P Q G G G H W S    421
V    422   Q V Q T I H G T Q I G S Y F G G E L C G V - D V D Q D G E T E L L L I G A P I F Y G E Q R G G R V F I Y Q R R Q L G F E F V S    483
VI   484   E L Q G D P G Y P L G R - F G E A I T A L T D I N G D G L V D - V A V G A P L E E Q - - - - G A V Y I F N G R H G G L S P Q P S Q    542
VII  543   R I E G T Q V L S G I Q W F G R S I H G V K D L E G D G L A D - V A V G A E S - Q M I V L S S R P V V D M V T L M S F S P A E I    604

            divalent cation binding site consensus        D - D G D G - I D - - E
                                                                   N

            divalent cation coordination sites            +X  +Y  +Z  -Y  -X    -Z

            divalent cation binding sites

                Parvalbumin    CD      51        D E D K S G F I E E D E
                               EF      90        D S D G D G K I G V D E

                Troponin C             27        D A D G G G D I S V K E
                                       63        D E D G S G T I D F E E
                                       103       D R N A D G Y I D P E E
                                       139       D K N N D G R I D F D E

                Galactose Binding     134        D L N K D G Q I Q - I E*
                Protein


                              Figure 4

... insertion of 200 residue I domain in LFA-1, Mac-1, p150,95 ...

Figure 5

```
LFA1     130  VDLVFLFDGSMSLQFDEFQKILDFMKDVM---KELSNTSYQFAAVQFSISYKTEFDFSDYV
MAC1     133  SDIAFLIDGSGSIIPHLFRRMKEFVSTVMEQLKKSKT--LFSLMQASEEFRIHFTFKEFQ
P150     131  QDIVFLIDGSGSISSRNFATMMNFVRAVISQFQRPST--QFSLMQFSNKFQTHFTFEEFR
VWFA1    513  LDLVFLLDGSSRLSEAEFEVLKAFVVDMMERLRI-SQKWVR--VAVVEYHDGSHAYIGLK
VWFA2    734  LDVAFVLEGSDKIGEADFNRSKEFMEEVIQRMDV-GQDSIH--VTVLQYSYMVTVEYPFS
VWFA3    927  LDVILDLDGSSSFPASYFDEMKSFAKAFISKANIGPRLTQ---VSVLQYGSITTIDVPWN
FACTOR B 244  MNIYLVLDGSDSIGASNFTGAKKCLVNLIEKVASYGVKPRYGLVTYATYPKIWVKVSEAD
CMP2     195  LDLVFLIDGSKSVRFENFELVKKFINQ-IVESLEV--SEKQFQVGLVQYSSSV-RQFPL
CMP1       1  -------------------------------------------VGVINYASAV-KNEFSL

LFA1          EMKLPDALLRHVKHMLLL-------TNTFGAI-NYVATEVFREELGARPDATKVL----L
MAC1          NNFNPRSLVKPITQLLGR-------IHTATGIT-NVVRELFNITNGARKNAFKIL----V
P150          RTSNPLSLLASVHQLQGF-------TYTATAIQ-NVVHRLFHASYGARRDATKIL----L
VWFA1         DPSRPSELRRIASQVKYAG------SQVQSTSEVLKYTLFQIFSKIDRPEASRIA---LL
VWFA2         EAQSKGDILQRRVREIRYQGGNR--TNTGLAL-RYLSDHSFLVSQGDREQAPNLV----Y
VWFA3         VVPEKAHLLSLVDVMQREGGPSQIGDALGFAV-RYLTSE------GARPGASKAV---VI
FACTOR B      SS-NADWVTRQLNEINYEDHKLKSGTNTKKALQAVYSMMSWPDDVPPEGWNRTRH--VII
CMP2          GQFKNKKDIKAA-VKKMAYMEG---IMTGQAL-KFLVDSSFSIANGARPGVPKVG----I
CMP1          ETHQTKFE-LLQAVARIEPLSTG--IMTGLAI-QFAISRAFSDTEGARLRSPNINKVAI-

LFA1          IITDGE-ATDSGNIDA---AKL--IIRYIIGIGKHFQTKESQET-LHKFASKPASEFVK
MAC1          VITDGEKFGDPLGYEDVIPEADREGVIRYVIGVGDAFRSENSRQE-LNTIASKPPRDHVF
P150          VITDGKKEGDSLDYKDVIPMADAAGIIRYAIGVGLAFQNRNSWKE-LNDIASKPSQEHIF
VWFA1         LMASQEPQRMSRNFVRYVQGLKKKKVIVIPVGIGPHANLKQIR---LIEKQAPENKAFV
VWFA2         MVTGNPASDEIKRL-PGDIQ------VVPIGVGPNANVQE-----LERIGWPNAP---
VWFA3         LVTDVSVDSVDAAADAARS---NRVTVFPIGIGDRYDAAQ-----LRILAG-PAGDSNV
FACTOR B      LMTDGLHNMGGDPITVIDE------IRDLLIIGNDRKNPR-EDYLDVYVFGVGP-LVN
CMP2          VFTDGRSQDYIT---DAAKKAKDLGFRMFAVGVGNAV------EDELREIASEPVAEHYF
CMP1          VVTDGRPQDGVQ-VSARARQAGIEIF---AIGVGRVD------MHTLRQIASEPLDDHVD

LFA1          IILDT-FEKLKDLFTELQKKIYVIEGTSKQDLTSFNMELSSS          327
MAC1          QVNN-FEALKTIQNQLREKIFAIEGTQTGSSSSFEHEMSQE          337
P150          KVED-FDALKDIQNQLKEKIFAIEGTETTSSSSFELEMAQE          335
VWFA1         LSS-VDELEQQRDEIVSTLCDLAPEAPPPTLPPDMAQVTVGP          717
VWFA2         IIIQDFETLPREAPLDVLQRC-CSGEGLQIPTLSPAPD          922
VWFA3         VKLQRIEDLPTMVTLGN-------SFLHKLCSGFVRI          1115
FACTOR B      QVNINALASK-KDNEQHVFK-VKDMENLEDVFYQMIDESQSL          450
CMP2          YTA-DFRTISNIGKNLQMKICVEEDPCECKSIVKFQTKV          393
CMP1          YVESYSVIEKLTHKGFDEAFCVVSDLCATGDHDCEQICI          172
```

Figure 6

Figure 7